# FASCICULE DE BREVET EUROPEEN

(11) **EP 1 326 705 B1**
(45) Date de publication et mention de la délivrance du brevet: **12.06.2013**
(21) Numéro de dépôt: 01980621.5
(22) Date de dépôt: 22.10.2001
(51) Int. Cl.: B01J 2/30, A61K 8/02, A61K 8/06, A61K 8/90, A61K 9/16

(54) **GRANULES OBTENUS PAR SECHAGE D'UNE EMULSION MULTIPLE**
DURCH TROCKNUNG VON MEHRFACHEN EMULSIONEN HERGESTELLTE TEILCHEN
GRANULES OBTAINED BY DRYING A MULTIPLE EMULSION

(30) Priorité: 20.10.2000 FR 0013466
(43) Date de publication de la demande: 16.07.2003
(73) Titulaire: RHODIA CHIMIE, 92512 Boulogne Billancourt Cedex (FR)
(72) Inventeur: LANNIBOIS-DREAN, Hélène, F-94220 Charenton le Pont (FR); MORVAN, Mikel, F-92600 Asnières (FR); TAISNE, Laurent, F-75018 Paris (FR)
(74) Mandataire: Delenne, Marc
(86) Numéro de dépôt international: PCT/FR2001/003272
(87) Numéro de publication internationale: WO 2002/032563

(56) Documents cités:
- FR-A- 2 785 198
- US-A- 4 954 298
- US-A- 5 750 124
- US-A- 5 840 943
- "Ullmann's Encyclopedia of Industrial Chemistry. 5th Edition. Volume A9" 1988 , VCH , WEINHEIM (ALLEMAGNE) XP002192946 page 321 -page 325

## Description

La présente invention a pour objet des granulés susceptibles d'être obtenus par séchage d'une émulsion inverse dispersée dans une phase aqueuse.

Les émulsions multiples, ou plus particulièrement les émulsions inverses (eau dans huile) dispersées dans une phase aqueuse, sont un moyen très intéressant si l'on veut, dans une même formulation, introduire deux matières actives hydrophiles incompatibles. Le développement de formulations complexes, type "deux en un", fait que l'on se retrouve de plus en plus souvent confronté à ces problèmes d'incompatibilité. Il n'est pas rare en effet de souhaiter, lors de l'application, avoir dans un mélange, deux substances qui, lorsqu'elles se trouvent en contact, réagissent entre elles. L'utilisation d'émulsions multiples peut aussi devenir essentielle lorsque la formulation comprend plusieurs matières actives qu'il faut conserver isolées car une fois mises en présence, l'une d'elles dégrade l'autre par exemple. Ainsi, dans de nombreux cas de figures, il est nécessaire de faire en sorte que les substances actives entrent en contact seulement lors de l'utilisation de la formulation qui les renferme, sous peine de rendre cette dernière inefficace ou inutilisable. US-A-4954298 décrit l'utilisation des émulsions multiples pour préparer des microcapsules.

Les emulsions multiples peuvent de même apporter une solutionappropriée si, dans une formulation comprenant plusieurs matières actives, on veut conférer un effet retard à l'une d'entre elles. En effet, la matière active se trouvant dans l'émulsion inverse de l'émulsion multiple ne sera pas disponible aussi rapidement que celle se trouvant dans la phase externe aqueuse de l'émulsion multiple.

Le problème des émulsions multiples est que ce sont des systèmes qui se trouvent hors équilibre, ce qui signifie qu'ils sont relativement instables. Des moyens sont connus pour remédier à ces problèmes d'instabilité mais ils ne sont que temporaires. En effet, s'ils apportent une solution relativement satisfaisante pour conférer une stabilité suffisante de cette émulsion durant le temps de préparation de la formulation la comprenant, ils ne permettent toutefois pas de garantir la stabilité l'émulsion multiple au cours du stockage.

La présente invention a donc pour objet de proposer une composition stable au stockage, dérivant d'une émulsion multiple et qui puisse être, si cela est souhaité, redispersée dans un milieu aqueux sous la forme d'une émulsion multiple.

L'invention consiste donc en des granulés susceptibles d'être obtenus par séchage d'une émulsion inverse, dispersée dans une phase aqueuse externe, ledit séchage éliminant la phase aqueuse externe,
(a) l'emulsion inverse comprenant une phase aqueuse interne comprenant au moins une une matière active hydrophile, dispersée dans une phase organique interne, ladite émulsion inverse comprenant au moins un tensioactif non ionique et/ou au moins un polymère amphiphile à blocs, et/ou au moins un tensioactif cationique ;
(b) la phase aqueuse externe comprenant :
   - au moins un tensioactif polyalcoxylé non ionique et/ou au moins un polymère amphiphile polyalcoxylé non ionique,
   - au moins un polymère hydrosoluble ou hydrodispersable se présentant sous une forme solide en présence d'une teneur en eau d'au plus 10 % en poids par rapport au poids dudit polymère et dont la température de transition vitreuse est supérieure à 25°C, de préférence supérieure à 50°C.

Dans ce qui va suivre, le terme "granulés" désigne des particules dont la taille moyenne varie de 100 µm à quelques millimètres.

Comme cela a été indiqué auparavant, les granulés selon l'invention présentent l'avantage d'être stables au stockage et de donner à nouveau, après redispersion dans une phase aqueuse, une émulsion multiple.

Il est à noter qu'il est tout à fait surprenant de constater que l'opération de séchage, consistant à éliminer la phase aqueuse externe de l'émulsion multiple, n'a pas eu pour effet d'éliminer totalement l'eau des gouttelettes de l'émulsion inverse. Effectivement, l'homme de l'art s'attendait à avoir un phénomène de déstabilisation de l'émulsion multiple durant l'étape de séchage, ce qui aurait abouti inévitablement à l'obtention d'une émulsion simple après redispersion dans une phase aqueuse des granulés issus du séchage. Or contrairement à ce que l'on était en droit d'attendre, une émulsion multiple a pu être à nouveau obtenue par dispersion dans un milieu aqueux, des granulés issus du séchage d'une telle émulsion.

Parmi d'autres avantages des granulés obtenus par séchage d'une émulsion multiple, on peut citer celui de pouvoir conserver une matière active, sous la forme d'une solution ou d'une dispersion aqueuse, dispersée dans une composition qui a l'aspect d'un solide divisé. De tels granulés permettent de conserver des substances nécessitant la présence d'eau pour être actives ou actives sans temps de latence trop important. C'est notamment le cas des bactéries, des enzymes ou encore de certains sels hydratés.

Mais d'autres buts et avantages apparaîtront plus clairement à la lecture de la description et de l'exemple, qui vont suivre.

Dans la description, le terme polymère désigne à la fois des homopolymères et des copolymères.

De plus, le terme macromonomère désigne une macromolécule portant une ou plusieurs fonctions susceptibles d'être polymérisées, notamment par voie radicalaire.

Pour des raisons de commodité dans l'exposé de l'invention, l'émulsion inverse et son mode de préparation vont tout d'abord être détaillés.

L'émulsion inverse consiste donc en une émulsion eau dans huile. Par la suite, la phase aqueuse de cette émulsion sera appelée phase aqueuse interne, et la phase organique, phase organique interne.

Le composé utilisé en tant que phase organique est de préférence choisi parmi les composés dont la solubilité dans l'eau ne dépasse pas 10 % en poids à 25°C.

En outre, ledit composé est choisi parmi ceux qui ne sont pas éliminés dans les conditions de séchage de l'émulsion multiple.

En tant que composé convenable, on peut citer notamment les huiles organiques, d'origine animale ou végétale, ou minérales, ainsi que les cires provenant des mêmes origines, ou leurs mélanges.

Comme huiles organiques d'origine animale, on peut citer entre autres, l'huile de cachalot, l'huile de baleine, l'huile de phoque, l'huile de sardine, l'huile de hareng, l'huile de squale, l'huile de foie de morue ; les graisses de porc, de mouton (suifs).

En tant que cires d'origine animale, on peut citer la cire d'abeille.

A titres d'exemples d'huiles organiques d'origine végétale, on peut mentionner, entre autres, l'huile de colza, l'huile de tournesol, l'huile d'arachide, l'huile d'olive, l'huile de noix, l'huile de maïs, l'huile de soja, l'huile de lin, l'huile de chanvre, l'huile de pépins de raisin, l'huile de coprah, l'huile de palme, l'huile de graines de coton, l'huile de babassu, l'huile de jojoba, l'huile de sésame, l'huile de ricin, le beurre de cacao, le beurre de karité.

En tant que cires d'origine végétale, on peut citer la cire de carnauba.

En ce qui concerne les huiles minérales, on peut citer entre autres les coupes pétrolières, les huiles naphténiques, paraffiniques (vaseline). Les cires paraffiniques peuvent de même convenir à la préparation de l'émulsion.

Les produits issus de l'alcoolyse des huiles précitées peuvent aussi être utilisés.

On ne sortirait du cadre de la présente invention en mettant en oeuvre, en tant que phase organique interne, au moins un acide gras, saturé ou non, au moins un alcool gras, saturé ou non, au moins un ester d'acide gras, ou leurs mélanges.

Plus particulièrement, lesdits acides comprennent 8 à 40 atomes de carbone, plus particulièrement 10 à 40 atomes de carbone, de préférence 18 à 40 atomes de carbone, et peuvent comprendre une ou plusieurs insaturations éthyléniques, conjuguées ou non, et éventuellement un ou plusieurs groupements hydroxyles. Quant aux alcools, ils peuvent comprendre un ou plusieurs groupements hydroxyles.

Comme exemples d'acides gras saturés, on peut citer les acides palmitique, stéarique, béhénique.

Comme exemples d'acides gras insaturés, on peut citer les acides myristoléique, palmitoléique, oléique, érucique, linoléique, linolénique, arachidonique, ricinoléique, ainsi que leurs mélanges.

Quant aux alcools, ceux-ci comprennent plus particulièrement 4 à 40 atomes de carbone, de préférence 10 à 40 atomes de carbone, éventuellement une ou plusieurs insaturations éthyléniques, conjuguées ou non, et éventuellement plusieurs groupements hydroxyles. Les polymères comprenant plusieurs groupement hydroxyles peuvent de même convenir, comme par exemple les polypropylèneglycols.

Comme exemple d'alcools, on peut citer par exemple ceux correspondants aux acides précités.

Concernant les esters d'acides gras, ceux-ci peuvent avantageusement être obtenus à partir d'acides gras, choisis parmi les composés nommés ci-dessus. Les alcools à partir desquels ces esters sont préparés comprennent plus particulièrement 1 à 6 atomes de carbone. De préférence, il s'agit d'esters méthylique, éthylique, propylique, isopropylique.

Par ailleurs, il n'est pas exclu d'utiliser en tant que phase organique des mono-, di- et tri- glycérides.

Enfin, la phase organique peut comprendre une quantité d'eau qui ne dépasse pas la limite de solubilité de l'eau dans ladite phase organique (à une température comprise entre 20 et 30°C).

La phase organique interne peut de même être choisie parmi les résines alkydes (comme par exemple les résines Coporob 3115 DE, commercialisées par la société Novance), les résines époxy, les (poly)isocyanates masqués ou non.

Elle peut aussi être choisie parmi les huiles essentielles ainsi que les huiles silicones.

La phase organique interne peut comprendre au moins une matière active hydrophobe, dès l'instant qu'elle est compatible avec la matière active hydrophile présente dans la phase aqueuse interne, qui sera décrite par la suite.

Lesdites matières actives se présentent sous forme liquide ou non, soluble dans la phase organique ou solubilisée dans un solvant organique miscible dans la phase organique, ou encore sous la forme d'un solide en dispersion dans ladite phase.

Dans le cas où un solvant organique est présent, il est de préférence choisi parmi les substances qui ne sont pas éliminées dans les conditions de séchage de l'émulsion.

Plus particulièrement, les matières actives sont telles que leur solubilité dans l'eau ne dépasse pas 10 % en poids à 25°C.

En outre, les matières actives présentent de préférence une température de fusion inférieure ou égale à 100°C, plus particulièrement inférieure ou égale à 80°C.

Il est de même précisé que les matières actives organiques sont avantageusement choisies parmi les composés qui ne sont pas éliminés dans les conditions de séchage de l'émulsion multiple.

A titre d'exemple de matières actives dans le domaine de l'alimentaire, on peut citer les mono-, di- et triglycérides, les huiles essentielles, les arômes, les colorants.

A titre d'exemple de matières actives dans le domaine de la cosmétique on peut citer les huiles silicones appartenant par exemple à la famille des diméthicones ; les vitamines lipophiles, comme la vitamine A.

A titre d'exemple de matières actives convenables pour la réalisation de l'invention, dans le domaine des peintures, on peut citer les résines alkydes, les résines époxy, les (poly)isocyanates masqués ou non.

Dans le domaine du papier, on peut citer à titre d'exemples les résines de collage et d'hydrofugation, telles que le dimère d'alkylcétène (AKD) ou l'anhydride alcényle succinique (ASA).

Dans le domaine de l'agrochimie, les matières actives phytosanitaires peuvent être choisies parmi la famille des α-cyano-phénoxybenzyl carboxylates ou des α-cyano-halogénophénoxy-carboxylates, la famille des N-méthylcarbonates comprenant des substituants aromatiques, les matières actives telles que Aldrin, Azinphos-methyl, Benfluralin, Bifenthrin, Chlorphoxim, Chlorpyrifos, Fluchloralin, Fluroxypyr, Dichlorvos, Malathion, Molinate, Parathion, Permethrin, Profenofos, Propiconazole, Prothiofos, Pyrifenox, Butachlor, Metolachlor, Chlorimephos, Diazinon, Fluazifop-P-butyl, Heptopargil, Mecarbam, Propargite, Prosulfocarb, Bromophos-ethyl, Carbophenothion, Cyhalothrin.

Dans le domaine de la détergence, on peut mentionner en tant que matières actives possibles les antimousses silicones.

Il est de même possible d'utiliser des matières actives telles que celles entrant dans la composition de lubrifiants pour le travail ou la déformation des matériaux. La matière active est habituellement une huile, un dérivé d'une huile ou encore un ester d'acide gras.

La matière active peut aussi être choisie parmi les solvants organiques ou les mélanges de tels solvants pas ou peu miscibles dans l'eau, comme notamment ceux mis en oeuvre pour le nettoyage ou le décapage, tels que les coupes pétrolières aromatiques, les composés terpéniques comme les D- ou L- limonènes, ainsi que les solvants comme le Solvesso^{®}. Conviennent aussi comme solvants, les esters aliphatiques, comme les esters méthyliques d'un mélange d'acides acétique, succinique et glutarique (mélange d'acides sous-produit de la synthèse du Nylon), les huiles hydrocarbonées comme l'huile de vaseline, et les solvants chlorés.

Au cas où la phase organique interne comprend une ou plusieurs matières actives hydrophobes différentes de la phase organique, leur teneur représente plus particulièrement 10 à 50 % en poids de ladite phase organique interne.

Enfin, la phase organique elle-même peut être considérée comme matière active hydrophobe.

L'émulsion inverse comprend en outre au moins un tensioactif non ionique et/ou au moins un polymère amphiphile à blocs, et/ou au moins un tensioactif cationique.

Selon une première variante, l'émulsion inverse comprend au moins un tensioactif non ionique ou au moins un polymère amphiphile à blocs, ou un mélange d'entre eux.

Il est à noter que la règle de Bancroft peut être appliquée au tensioactif non ionique et au polymère amphiphile à blocs utilisés (2^{éme} Congrès Mondial de l'Emulsion, 1997, Bordeaux, France). En d'autres termes, la fraction soluble dans la phase continue est supérieure à la fraction soluble dans la phase dispersée.

Ainsi, le tensioactif et le polymère sont de préférence choisis parmi ceux qui vérifient à la fois les deux conditions ci-dessous :
- lorsqu'ils sont mélangés avec la phase organique interne, à une concentration comprise entre 0,1 et 10 % en poids de ladite phase à 25°C, se trouvent sous la forme d'une solution dans tout ou partie de la gamme de concentration indiquée.
- lorsqu'ils sont mélangés avec la phase aqueuse interne, à une concentration comprise entre 0,1 et 10 % en poids de ladite phase et à 25°C, se trouvent sous la forme d'une dispersion dans tout ou partie de la gamme de concentration indiquée.

Plus particulièrement, le tensioactif non ionique est choisi parmi les composés présentant une valeur de HLB (balance hydrophile/lipophile) inférieure ou égale à 8.

A titre d'exemples de tensioactifs susceptibles d'entrer dans la composition de l'émulsion inverse, on peut citer les tensioactifs, seuls ou en mélange, choisis parmi :
- les alcools gras alcoxylés
- les triglycérides alcoxylés
- les acides gras alcoxylés
- les esters de sorbitan éventuellement alcoxylés
- les amines grasses alcoxylées
- les di(phényl-1 éthyl) phénols alcoxylés
- les tri(phényl-1 éthyl) phénols alcoxylés
- les alkyls phénols alcoxylés
le nombre de motifs alcoxylés (éthoxylés, propoxylés, butoxylés) est tel que la valeur de HLB soit inférieure ou égale à 8.

Les alcools gras alcoxylés comprennent généralement de 6 à 22 atomes de carbone, les motifs alcoxylés étant exclus de ces nombres.

Les triglycérides alcoxylés peuvent être des triglycérides d'origine végétale ou animale.

Les esters de sorbitan éventuellement alcoxylés sont des esters du sorbitol cyclisés d'acide gras comprenant de 10 à 20 atomes de carbone comme l'acide laurique, l'acide stéarique ou l'acide oléïque.

Les amines grasses alcoxylées ont généralement de 10 à 22 atomes de carbone, les motifs alcoxylés étant exclus de ces nombres.

Les alkylphénols alcoxylés ont généralement un ou deux groupes alkyles, linéaires ou ramifiés, ayant 4 à 12 atomes de carbone. A titre d'exemple on peut citer notamment les groupes octyle, nonyle ou dodécyle.

Quant au polymère amphiphile à blocs, celui-ci comprend au moins deux blocs.

Ces polymères amphiphiles, vérifiant la règle de Bancroft et les deux conditions énoncées auparavant, comprennent plus particulièrement au moins un bloc hydrophobe et au moins un bloc hydrophile neutre, anionique ou cationique.

Au cas où le polymère amphiphile comprend au moins trois blocs, et plus particulièrement trois blocs, le polymère est de préférence linéaire. En outre, les blocs hydrophobes se trouvent plus particulièrement aux extrémités.

Au cas où les polymères comprennent plus de trois blocs, ces derniers sont de préférence sous la forme de polymères greffés ou peignes.

Dans ce qui suit, même si cela constitue un abus de langage, le terme polymère amphiphile à blocs sera utilisé indifféremment pour les polymères linéaires à blocs et les polymères greffés ou peignes.

Lesdits polymères amphiphiles peuvent de manière avantageuse, être obtenus par polymérisation radicalaire dite vivante ou contrôlée. A titre d'exemples non limitatifs de procédés de polymérisation dite vivante ou contrôlée, on peut notamment se référer aux demandes WO 98/58974 (xanthate), WO 97/01478 (dithioesters), WO 99/03894 (nitroxydes) ; WO 99/31144 (dithiocarbamates).

Les polymères amphiphiles peuvent aussi être obtenus par polymérisation cationique, anionique.

Ils peuvent de même être préparés en mettant en jeu des polymérisations par ouverture de cycle (notamment anionique ou cationique), ou par modification chimique du polymère.

Les polymères greffés ou peignes peuvent encore être obtenus par des méthodes dites de greffage direct et copolymérisation.

Le greffage direct consiste à polymériser le(s) monomère(s) choisi(s) par voie radicalaire, en présence du polymère sélectionné pour former le squelette du produit final. Si le couple monomère/squelette ainsi que les conditions opératoires, sont judicieusement choisis, alors il peut y avoir réaction de transfert entre le macroradical en croissance et le squelette. Cette réaction génère un radical sur le squelette et c'est à partir de ce radical que croît le greffon. Le radical primaire issu de l'amorceur peut également contribuer aux réactions de transfert.

Pour ce qui a trait à la copolymérisation, elle met en oeuvre dans un premier temps le greffage à l'extrémité du futur segment pendant, d'une fonction polymérisable par voie radicalaire. Ce greffage peut être réalisé par des méthodes usuelles de chimie organique. Puis, dans un second temps, le macromonomère ainsi obtenu est polymérisé avec le monomère choisi pour former le squelette et on obtient un polymère dit "peigne".

Parmi les monomères hydrophobes à partir desquels le ou les blocs hydrophobes du polymère amphiphile peuvent être préparés, on peut citer notamment :
- les esters des acides mono- ou poly- carboxyliques, linéaires, ramifiés, cycliques ou aromatiques, comprenant au moins une insaturation éthylénique,
- les esters d'acides carboxyliques saturés comprenant 8 à 30 atomes de carbone, éventuellement porteurs d'un groupement hydroxyle ;
- les nitriles αβ-éthyléniquement insaturés, les éthers vinyliques, les esters vinyliques, les monomères vinylaromatiques, les halogénures de vinyle ou de vinylidène,
- les monomères hydrocarbonés, linéaires ou ramifiés, aromatiques ou non, comprenant au moins une insaturation éthylénique,
- les monomères de type siloxane cyclique ou non, les chlorosilanes ;
- l'oxyde de propylène, l'oxyde de butylène ;
seuls ou en mélanges, ainsi que les macromonomères dérivant de tels monomères.

A titre d'exemples particuliers de monomères hydrophobes susceptibles d'entrer dans la préparation du ou des blocs hydrophobes du polymère amphiphile à blocs, on peut citer :
- les esters d'acide (méth)acrylique avec un alcool comprenant 1 à 12 atomes de carbone comme le (méth)acrylate de méthyle, le (méth)acrylate d'éthyle, le (méth)acrylate de propyle, le (méth)acrylate de n-butyle, le (méth)acrylate de t-butyle, le (méth)acrylate d'isobutyle, l'acrylate de 2-éthylhexyl ;
- l'acétate de vinyle, le Versatate^{®} de vinyle, le propionate de vinyle, le chlorure de vinyle, le chlorure de vinylidène, le méthyl vinyléther, l'éthyl vinyléther ;
- les nitriles vinyliques incluent plus particulièrement ceux ayant de 3 à 12 atomes de carbone, comme en particulier l'acrylonitrile et le méthacrylonitrile ;
- le styrène, l'α-méthylstyrène, le vinyltoluène, le butadiène, le chloroprène ;
seuls ou en mélanges, ainsi que les macromonomères dérivant de tels monomères.

Les monomères préférés sont les esters de l'acide acryliques avec les alcools linéaires ou ramifiés en C₁-C₄ tels que l'acrylate de méthyle, d'éthyle, de propyle et de butyle, les esters vinyliques comme l'acétate de vinyle, le styrène, l'α-méthylstyrène.

En ce qui concerne les monomères hydrophiles non ioniques à partir desquels les polymères amphiphiles à blocs peuvent être obtenus, on peut mentionner, sans intention de s'y limiter, l'oxyde d'éthylène, les amides des acides mono- ou polycarboxyliques, linéaires, ramifiés, cycliques ou aromatiques, comprenant au moins une insaturation éthylénique ou dérivés, comme le (méth)acrylamide, le N-méthylol (méth)acrylamide ; les esters hydrophiles dérivant de l'acide (méth)acrylique comme par exemple le (méth)acrylate de 2-hydroxyéthyle ; les esters vinyliques permettant d'obtenir des blocs alcool polyvinylique après hydrolyse, comme l'acétate de vinyle, le Versatate^{®} de vinyle, le propionate de vinyle, seuls, en combinaison, ainsi que les macromonomères dérivant de tels monomères. Il est rappelé que le terme macromonomère désigne une macromolécule portant une ou plusieurs fonctions polymérisables.

Toutefois les monomères hydrophiles préférés sont l'acrylamide et le méthacrylamide, seuls ou en mélange, ou sous la forme de macromonomères.

En ce qui concerne les monomères hydrophiles anioniques à partir desquels les polymères amphiphiles à blocs peuvent être obtenus, on peut mentionner, par exemple les monomères comprenant au moins une fonction carboxylique, sulfonique, sulfurique, phosphonique, phosphorique, sulfosuccinique, ou les sels correspondants.

Il est précisé que dans les conditions de pH d'utilisation du polymère amphiphile à blocs, les fonctions du ou des blocs anioniques du polymère se trouvent sous une forme au moins partiellement ionisée (dissociée). Plus particulièrement, au moins 10 % en mole des fonctions du ou des blocs sont sous forme ionisée. La détermination de cette valeur ne pose pas de problème à l'homme de l'art ; elle est notamment fonction du pKa des fonctions ionisables des motifs du polymère et du nombre de ces fonctions (soit du nombre de moles de monomère portant des fonctions ionisables mis en oeuvre lors de la préparation du polymère).

Plus particulièrement, les monomères sont choisis parmi :
- les acides mono- ou poly- carboxyliques linéaires, ramifiés, cycliques ou aromatiques, les dérivés N-substitués de tels acides ; les monoesters d'acides polycarboxyliques, comprenant au moins une insaturation éthylénique ;
- les acides vinyl carboxyliques linéaires, ramifiés, cycliques ou aromatiques ;
- les aminoacides comprenant une ou plusieurs insaturations éthyléniques ;
seuls ou en mélanges, leurs précurseurs, leurs dérivés sulfoniques ou phosphoniques, ainsi que les macromonomères dérivant de tels monomères ; les monomères ou macromonomères pouvant être sous la forme de sels.

A titre d'exemples de monomères anioniques, on peut citer sans intention de s'y limiter :
- l'acide acrylique, l'acide méthacrylique, l'acide fumarique, l'acide itaconique, l'acide citraconique, l'acide maléique, l'acide acrylamido glycolique, l'acide 2-propène 1-sulfonique, l'acide méthallyl sulfonique, l'acide styrène sulfonique, l'acide α-acrylamido méthylpropane sulfonique, le 2-sulfoéthylène méthacylate, l'acide sulfopropyl acrylique, l'acide bis-sulfopropyl acrylique, l'acide bis-sulfopropyl méthacrylique, l'acide sulfatoéthyl méthacrylique, le monoester phosphate d'acide hydroxyéthyl méthacrylique, ainsi que les sels de métal alcalin, comme le sodium, le potassium, ou d'ammonium ;
- l'acide vinyl sulfonique, l'acide vinylbenzène sulfonique, l'acide vinyl phosphonique, l'acide vinylidène phosphorique, l'acide vinyl benzoïque, ainsi que les sels de métal alcalin, comme le sodium, le potassium, ou d'ammonium ;
- le N-méthacryloyl alanine, le N-acryloyl-hydroxy-glycine ;
seuls ou en mélanges, ainsi que les macromonomères dérivant de tels monomères.

On ne sortirait pas du cadre de la présente invention en mettant en oeuvre des monomères précurseurs de ceux qui viennent d'être cités. En d'autres termes, ces monomères présentent des motifs qui, une fois incorporés dans la chaîne polymère, peuvent être transformés, notamment par un traitement chimique tel que l'hydrolyse, pour redonner les espèces anioniques précitées. Par exemple, les monomères totalement ou partiellement estérifiés des monomères précités peuvent être mis en oeuvre pour être, par la suite, hydrolysés totalement ou en partie.

En tant que monomères hydrophiles cationiques à partir desquels les polymères amphiphiles à blocs peuvent être obtenus, on peut citer notamment :
- les (méth)acrylates d'aminoalkyle, les (méth)acrylamides d'aminoalkyle ;
- les monomères comprenant au moins une fonction amine secondaire, tertiaire ou quaternaire, ou un groupe hétérocyclique contenant un atome d'azote, la vinylamine, l'éthylène imine ;
- les sels d'ammonium de diallyldialkyl ;
seuls ou en mélanges, ou les sels correspondants, ainsi que les macromonomères dérivant de tels monomères.

Lesdits monomères peuvent présenter un contre-ion choisi parmi les halogénures comme par exemple le chlore, les sulfates, les hydrosulfates, les alkylsulfates (par exemple comprenant 1 à 6 atomes de carbone), les phosphates, les citrates, les formates, les acétates.

A titre d'exemples de monomères cationiques convenables figurent entre autres les monomères suivants :
- diméthyl amino éthyl (méth)acrylate, diméthyl amino propyl (méth)acrylate le ditertiobutyl aminoéthyl (méth)acrylate, le diméthyl amino méthyl (méth)acrylamide, le diméthyl amino propyl (méth)acrylamide ;
- l'éthylène imine, la vinylamine, la 2-vinylpyridine, la 4-vinylpyridine ;
- le chlorure de triméthylammonium éthyl (méth)acrylate, le méthyl sulfate de triméthylammonium éthyl acrylate, le chlorure de benzyl diméthylammonium éthyl (méth)acrylate, le chlorure de 4-benzoylbenzyl diméthyl ammonium éthyl acrylate, le chlorure de triméthyl ammonium éthyl (méth)acrylamido, le chlorure de triméthyl ammonium de vinylbenzyle ;
- le chlorure d'ammonium de diallyldiméthyl ;
seuls ou en mélanges, ou leurs sels correspondants, ainsi que les macromonomères dérivant de tels monomères.

De préférence, les polymères amphiphiles à blocs présentent une masse molaire en poids inférieure ou égale à 100000 g/mol, plus particulièrement comprise entre 1000 et 50000 g/mol, de préférence entre 1000 et 20000 g/mol. Il est précisé que les masses molaires en poids indiquées ci-dessus sont des masses molaires théoriques, évaluées en fonction des quantités respectives des monomères introduites lors de la préparation desdits polymères.

De préférence, on met en oeuvre un polymère amphiphile à bloc de type non ionique.

A titre d'exemple de polymère amphiphile à blocs convenant à la mise en oeuvre de l'invention, on peut citer les polymères triblocs polyhydroxystéarate - polyéthylène glycol - polyhydroxystéarate (les produits de la gamme Arlacel de ICI en sont un exemple), les polymères à blocs polydiméthylsiloxane greffé polyéther polyalkyle (comme les produits de la marque Tegopren commercialisé par Goldschmidt).

Selon une deuxième variante, l'émulsion inverse comprend au moins un tensioactif cationique.

Dans le cas de cette variante, il est indiqué que le tensioactif cationique ne vérifie pas la règle de Bancroft énoncée auparavant. En effet, le tensioactif cationique est soluble dans la phase dispersée et non dans la phase continue de l'émulsion inverse.

Parmi les tensioactifs cationiques convenables, on peut notamment utiliser les amines grasses aliphatiques ou aromatiques, les amides gras aliphatiques, les dérivés d'ammonium quaternaire (Rhodaquat RP50 de Rhodia Chimie).

Enfin, une troisième variante de l'invention consiste à combiner les deux possibilités qui viennent d'être détaillées.

Quelle que soit la variante retenue, la teneur totale en tensioactif non ionique, en polymère amphiphile à blocs et/ou en tensioactif cationique représente plus particulièrement de 0,1 à 10 % en poids, de préférence de 2 à 10 % en poids par rapport à la phase aqueuse interne.

La phase aqueuse interne comprend au moins une matière active hydrophile, se présentant sous une forme soluble dans la phase aqueuse interne ; sous une forme solubilisée dans un solvant miscible à l'eau comme le méthanol, l'éthanol, le propylène glycol, le glycérol ; sous la forme d'un solide en dispersion dans ladite phase.

La quantité de matière active hydrophile, si elle est présente, l'est à une teneur plus particulièrement comprise entre 0,1 et 50 % en poids de la phase aqueuse interne, et de préférence comprise entre 0,1 et 20 % en poids de la phase aqueuse interne.

Etant donné le nombre important de domaines dans lesquels les granulés selon l'invention sont susceptibles d'être utilisés, de nombreuses matières actives peuvent convenir à la mise en oeuvre de l'invention.

A titre d'exemple de matières actives utilisables dans le domaine de la cosmétique, on peut citer les substances qui ont un effet cosmétique, un effet thérapeutique ou tout autre substance utilisable pour le traitement de la peau et du cheveu.

Ainsi, on peut utiliser, en tant que matière active des agents conditionneurs de la peau et du cheveu, comme notamment les polymères comprenant des ammonium quaternaires qui peuvent éventuellement être engagés dans des hétérocycles (composés du type des quaternium, polyquaternium, etc.)., des agents humectants ; des agents fixants (styling) qui sont plus particulièrement choisis parmi des polymères (homo-, co- ou ter-polymères par exemple acrylamide, acrylamide/acrylate de sodium, polystyrène sulfonate, etc.), les polymères cationiques, la polyvinylpyrrolidone, l'acétate de polyvinyle,etc.

Il est de même possible d'utiliser des agents colorants ; des agents astringents, utilisables dans les déodorants et qui sont plus particulièrement des sels d'aluminium, de zirconium ; des agents antibactériens ; des agents anti-inflammatoires, des agents anesthésiants, des filtres solaires, etc.

On peut aussi citer les α- et β- hydroxyacides, comme les acides citrique, lactique, glycolique, salicylique ; les acides dicarboxyliques de préférence insaturés et comprenant 9 à 16 atomes de carbone comme l'acide azélaique ; la vitamine C et ses dérivés, notamment les dérivés glycosylés et phosphatés ; les biocides notamment cationiques (Glokill PQ, Rhodoaquat RP50, commercialisés par Rhodia Chimie), comme matières actives convenables dans les formulations cosmétiques.

Dans le domaine de l'alimentaire, on peut citer par exemple les sels divalents de calcium (les phosphates, les chlorures, etc.) utilisés comme agent réticulant de polymères texturant comme les alginates, les carraghénannes ; le bicarbonate de sodium, entre autres.

Dans le domaine des matières actives phytosanitaires, on peut utiliser des pesticides hydrophiles ou des éléments nutritifs hydrophiles favorisant la croissance et le développement des plantes.

Pour ce qui a trait au domaine de l'exploitation ou la construction de puits de gisement de pétrole ou de gaz, la présente invention peut être mise en oeuvre pour des matières actives hydrophiles utilisables notamment lors des opérations cimentation, complétion, forage et stimulation des puits (par exemple la fracturation). A titre d'exemples de matières actives utilisables dans ce domaine, on peut mentionner des catalyseurs de réticulation de compositions cimentaires, comme par exemple les sels de lithium, comme le chlorure, l'acétate. On peut de même citer des composés susceptibles, entre autres, de dégrader les polysaccharides, comme par exemple les acides carboxyliques (notamment l'acide citrique), des enzymes (notamment les cellulases), des oxydants.

Dans le domaine des silicones, on peut citer par exemple les sels de calcium, la potasse, utilisés habituellement comme agents réticulants.

A titre de matières actives convenables dans le domaine de la fabrication du papier, on peut citer notamment le chlorure de calcium, l'acide chlorhydrique.

Conformément à un mode de réalisation particulièrement avantageux de la présente invention, la phase aqueuse interne peut comprendre au moins un additif choisi parmi les sels tels que les halogénures de métaux alcalins ou alcalino-terreux (comme le chlorure de sodium, le chlorure de calcium), ou les sulfates de métaux alcalin ou alcalino-terreux (comme le sulfate de calcium), ou leurs mélanges. La phase aqueuse interne peut aussi comprendre, en tant qu'additif, au moins un sucre, comme le glucose par exemple, ou encore au moins un polysaccharide, comme notamment le dextran, ou leurs mélanges.

La concentration en sel dans la phase aqueuse interne, lorsque ce dernier est présent, est plus particulièrement comprise entre 0,05 et 1 mol/l, de préférence 0,1 à 0,4 mol/l.

La concentration en sucre et/ou polysaccharide est telle que la pression osmotique de la phase aqueuse interne comprenant le sucre et/ou polysaccharide correspond à la pression osmotique d'une phase aqueuse interne comprenant 0,05 à 1 mol/l de sel.

En outre, l'émulsion inverse présente plus particulièrement une proportion pondérale phase aqueuse interne / phase organique interne comprise entre 10/90 et 90/10. De préférence, la proportion pondérale phase aqueuse / phase organique est comprise entre 30/70 et 80/20.

L'émulsion inverse est préparée en mettant en oeuvre les méthodes classiques.

Ainsi, pour ne citer qu'un exemple, on prépare, d'une part, un premier mélange comprenant l'eau, la matière active hydrophile, le tensioactif cationique s'il est présent, et éventuellement l'additif (sel, sucre et/ou polysaccharide) et d'autre part, un deuxième mélange comprenant le composé constituant la phase organique interne, éventuellement la matière active hydrophobe, et le tensioactif et/ou le polymère amphiphile à blocs, s'ils sont présents. On ajoute ensuite le premier mélange au second, sous agitation.

Dans le cas où la phase organique est peu visqueuse (viscosité inférieure à 5 Pa.s ; viscosité dynamique mesurée au Brookfield à 25°C, selon la norme NFT 76 102 de février 1972) l'agitation est de préférence forte et peut être, de manière avantageuse, apportée par l'utilisation d'un appareil du type Ultra-Turrax^{®}, Microfluidizer, ou tout homogénéisateur haute pression.

Dans le cas où la phase organique est visqueuse (viscosité supérieure ou égale à 5 Pa.s, mesurée comme précédemment), l'agitation peut être avantageusement effectuée au moyen d'une pale-cadre.

La préparation de l'émulsion inverse est en général réalisée à une température supérieure à la température de fusion du composé constituant la phase organique interne. Plus particulièrement, la température de préparation de l'émulsion inverse est comprise entre 20 et 80°C.

La durée de l'agitation peut être déterminée sans difficulté par l'homme de l'art et dépend du type d'appareillage mis en oeuvre. Elle est de préférence suffisante pour obtenir une taille moyenne de gouttelettes comprise entre 0,1 et 10 µm, de préférence entre 0,1 et 5 µm (mesurée au moyen d'un granulomètre Horiba). Il est rappelé que la taille moyenne des gouttelettes est mesurée au moyen d'un granulomètre Horiba, et correspond au diamètre médian en volume (d50) qui représente le diamètre de la particule égal à 50 % de la distribution cumulative.

La phase aqueuse externe de l'émulsion multiple va maintenant être décrite.

La phase aqueuse externe comprend au moins un tensioactif polyalcoxylé non ionique et/ou au moins un polymère amphiphile polyalcoxylé non ionique, et au moins un composé hydrosoluble ou hydrodispersable.

Là encore, la règle de Bancroft peut être appliquée au tensioactif et au polymère utilisés. En d'autres termes, la fraction soluble dans la phase continue est supérieure à la fraction soluble dans la phase dispersée.

Le tensioactif et le polymère vérifient donc simultanément, les deux conditions mentionnés ci-dessous :
- lorsqu'ils sont mélangés avec la phase aqueuse externe, à une concentration comprise entre 0,1 et 10 % en poids de ladite phase à 25°C, se trouvent sous la forme d'une solution dans tout ou partie de la gamme de concentration indiquée.
- lorsqu'ils sont mélangés avec la phase organique interne, à une concentration comprise entre 0,1 et 10 % en poids de ladite phase et à 25°C, se trouvent sous la forme d'une dispersion dans tout ou partie de la gamme de concentration indiquée.

De préférence, tensioactif polyalcoxylé non ionique présent dans la phase aqueuse externe présente une valeur de HLB supérieure ou égale à 10.

A titre d'exemple de tensioactif polyalcoxylé non ionique, convenant à la mise en oeuvre de l'invention, on peut citer entre autres, les tensioactifs suivants, seuls ou en mélanges :
- les alcools gras alcoxylés
- les triglycérides alcoxylés
- les acides gras alcoxylés
- les esters de sorbitan alcoxylés
- les amines grasses alcoxylées
- les di(phényl-1 éthyl) phénols alcoxylés
- les tri(phényl-1 éthyl) phénols alcoxylés
- les alkyls phénols alcoxylés.

Les motifs alcoxylés sont de préférence des motifs éthoxylés ou un mélange de motifs éthoxylés et propoxylés.

Les tensioactifs cités comme convenant à la préparation de l'émulsion inverse peuvent être repris, à l'exception du fait que le nombre de motifs éthoxylés et, s'ils sont présents, propoxylés, doivent être adaptés en fonction de la valeur de HLB souhaitée. A titre purement illustratif, le nombre de motifs éthoxylés et éventuellement propoxylés est compris entre 10 et 100.

Pour ce qui a trait au polymère amphiphile non ionique polyalcoxylé, ce dernier vérifie la règle de Bancroft et ses deux conditions énoncées auparavant, et comprend au moins deux blocs, l'un d'eux étant hydrophile, l'autre hydrophobe ; l'un au moins des blocs comprenant des motifs polyalcoxylés, plus particulièrement polyéthoxylés et/ou polypropoxylés.

Ce qui a été indiqué auparavant dans le cadre de la description des monomères hydrophiles non ioniques, des monomères hydrophobes utilisables pour la préparation des polymères amphiphiles à blocs entrant dans la composition de l'émulsion inverse, ainsi que des méthodes de synthèse, reste valable et ne sera pas repris ici.

A titre purement indicatif, lesdits polymères sont obtenus en mettant en jeu des polymérisations par ouverture de cycle, notamment anionique.

Plus particulièrement lesdits polymères amphiphiles polyalcoxylés non ioniques sont choisis parmi les polymères dont la masse molaire en poids est inférieure ou égale à 100000 g/mol (mesurée par GPC, étalon polyéthylène glycol), de préférence comprise entre 1000 et 50000 g/mol, de préférence comprise entre 1000 et 20000 g/mol.

A titre d'exemples de polymères de ce type, on peut citer entre autres les polymères triblocs polyéthylène glycol / polypropylène glycol / polyéthylène glycol. De tels polymères sont bien connus et sont notamment commercialisés sous les marques Pluronic (commercialisé par BASF), Arlatone (commercialisé par ICI).

Notons que l'on ne sortirait pas du cadre de la présente invention en combinant un ou plusieurs tensioactifs avec un ou plusieurs polymères amphiphiles.

Cependant, selon un mode de réalisation préféré de l'invention, la phase aqueuse externe comprend un ou plusieurs polymères amphiphiles.

La teneur en tensioactif polyalcoxylé non ionique et/ou en polymère amphiphile polyalcoxylé non ionique présent dans la phase aqueuse externe est plus particulièrement comprise entre 1 et 10 % en poids par rapport au poids de l'émulsion inverse. De préférence, la teneur en tensioactif non ionique et/ou en polymère amphiphile est comprise entre 1 et 5 % en poids par rapport au poids de l'émulsion inverse.

Il est à noter que si la phase organique interne présente une viscosité relativement élevée, par exemple supérieure ou égale à 5 Pa.s (viscosité dynamique mesurée au Brookfield à 25°C, selon la norme NFT 76 102 de février 1972), il peut être avantageux d'ajouter à la phase aqueuse externe, au moins un polymère thermoépaississant.

Les polymères thermoépaississants possèdent la particularité de donner des solutions aqueuses dont la viscosité augmente lorsque la température dépasse la température d'épaississement du polymère thermoépaississant ; température au delà de laquelle la viscosité du milieu dans lequel est présent ledit polymère augmente.

Plus particulièrement, ces polymères sont solubles dans l'eau à température ambiante, et au-delà de la température d'épaississement, une partie du polymère devient hydrophobe (partie thermosensible) : le polymère forme ainsi un réseau physique à l'échelle microscopique, ce qui se traduit à l'échelle macroscopique par une augmentation de la viscosité.

Selon un mode de réalisation avantageux de la présente invention, le polymère thermoépaississant mis en oeuvre est choisi parmi les polymères présentant un saut de viscosité entre 25 et 80°C tel que la valeur du rapport log₁₀ (viscosité à 80°C) /log₁₀ (viscosité à 25°C) est au moins égale à 1, de préférence au moins égale à 2.

Le rapport est mesuré dans les conditions suivantes :
Le polymère est tout d'abord mis en solution dans l'eau (extrait sec de 4 %).

Le profil rhéologique est ensuite mesuré en mode écoulement à contrainte imposée, en effectuant un balayage de température entre 20°C et 80°C. La configuration utilisée est la géométrie cône-plan 4cm/1 degré. La contrainte introduite dans le programme est choisie (en mode manuel) de telle sorte que le gradient à 25°C soit de 10 s⁻¹. La grandeur qui a été retenue pour caractériser le pouvoir thermoépaississant du polymère, soit le rapport log₁₀ (viscosité à 80°C) / log₁₀ (viscosité à 25°C), représente le saut de viscosité, exprimés en décades, de 25 à 80°C. Cette grandeur exprime en d'autres termes que la viscosité du milieu à 80°C est supérieure de 10ⁿ fois la viscosité du milieu à 25°C ; avec n entier compris entre 0 et 5.

Outre cette caractéristique, le polymère thermoépaississant est choisi de telle sorte que la variation de viscosité est réversible.

Parmi les polymères thermoépaississants utilisables, on peut citer les polysaccharides modifiés hydrophobes comme les carboxyméthyl celluloses, les méthyl celluloses, les hydroxyéthyl celluloses, les hydroxypropyl celluloses.

Dans le cas de ce type de polymères, il peut être avantageux de les mettre en oeuvre associés à au moins un tensioactif supplémentaire, choisi parmi les tensioactifs non ioniques ou encore anioniques.

Conviennent aussi les polymères synthétiques comme les polymères à base de N-isopropyl acrylamide, les polymères à base de N,N-diméthyl aminoéthyl méthacrylate.

Les polymères de structure peigne constitués d'un segment squelette polymérique sur lequel sont greffés au moins deux segments latéraux polymériques, identiques ou non, pour lequel soit le segment squelette polymérique, soit les segments latéraux polymériques possèdent une température critique inférieure de solubilité, LCST, comprise entre 25 et 80°C. De préférence, les segments latéraux polymériques sont thermosensibles et dérivent de polymères polyalcoxylés.

A titre d'exemples de polymères de ce type, on peut citer notamment, les polymères préparés à partir de polymère tri-blocs POE-POP-POE et d'acide acrylique (pourcentages molaires respectifs : 2,3 %, 97,7 %, greffage direct), les polymères préparés à partir de macromonomère de tri-blocs POE-POP-POE et d'acide acrylique (% molaires respectifs : 1,6 %, 98,4 %, copolymérisation), les polymères préparés à partir de macromonomère de tri-blocs POE-POP-POE et d'acide acrylique (% molaires respectifs : 3 %, 97 %, copolymérisation), les polymères préparés à partir de macromonomère de tri-blocs POE-POP-POE et d'acide acrylique (% molaires respectifs : 2 %, 98 %, copolymérisation).

Ces polymères ont fait l'objet d'une demande de brevet français FR 2 180 422, à laquelle on pourra se référer pour plus d'informations sur les polymères et leur obtention.

La teneur en polymère thermoépaississant représente plus particulièrement, lorsqu'il est présent, 0,2 à 10 % en poids de la phase aqueuse externe. De préférence, la teneur en ce polymère représente 1 à 5 % en poids de la phase aqueuse externe.

La phase aqueuse externe comprend par ailleurs au moins un composé hydrosoluble ou hydrodispersable se présentant sous une forme solide en présence d'une teneur en eau d'au plus 10 % en poids par rapport au poids dudit polymère et dont la température de transition vitreuse est supérieure à 25°C, de préférence supérieure à 50°C.

Par composé hydrosoluble ou hydrodispersable, on désigne un composé qui ne précipite pas lorsqu'il est en solution aqueuse, à 25°C, avec le tensioactif polyalcoxylé non ionique et/ou le polymère polyalcoxylé amphiphile non ionique présent(s) dans la phase aqueuse externe de l'émulsion multiple ; la concentration totale desdits tensioactif polyalcoxylé / polymère polyalcoxylé étant comprise entre 2 et 10 % en poids et la concentration en composé correspondant à une teneur comprise entre 30 et 85 % en poids dans le granulé final séché (et selon les variantes préférées qui seront explicitées plus loin, la concentration en composé correspondant à une teneur comprise entre 30 et 70 % en poids par rapport à la même référence, ou comprise entre 50 à 85 % en poids par rapport à la même référence).

Plus particulièrement, ledit composé hydrosoluble ou hydrodispersable est choisi parmi :
(i) au moins un polymère obtenu par polymérisation
   □ d'au moins un monomère (I) acide monocarboxylique ou polycarboxylique, où anhydride, aliphatique, cyclique ou aromatique, linéaire ou ramifié, éthyléniquement insaturé, et
   □ d'au moins un monomère hydrocarboné (II), linéaire ou ramifié, monoéthyléniquement ou polyéthyléniquement insaturé, et/ou
   □ d'au moins un monomère (III) ester polyalcoxylé d'acide carboxylique éthyléniquement insaturé ;
(ii) au moins un polymère issu de la polymérisation d'au moins un monomère (I) acide monocarboxylique ou polycarboxylique, ou anhydride, aliphatique, cyclique ou aromatique, linéaire ou ramifié, éthyléniquement insaturé, et comprenant éventuellement au moins un greffon hydrophobe, hydrocarboné, en C₄-C₃₀, saturé ou non, aromatique ou non, éventuellement interrompu par un ou plusieurs hétéroatomes ;
(iii) les protéines ou les polypeptides d'origine naturelle ou synthétique, comportant éventuellement au moins un greffon hydrophobe, hydrocarboné, en C₄-C₃₀, saturé ou non, aromatique ou non, éventuellement interrompu par un ou plusieurs hétéroatomes ;
(iv) les polysaccharides, de préférence fortement dépolymérisés comportant éventuellement au moins un greffon hydrophobe, hydrocarboné, en C₄-C₃₀, saturé ou non aromatique ou non, éventuellement interrompu par un ou plusieurs hétéroatomes;
(v) l'alcool polyvinylique, la polyvinylpyrrolidone.

Selon une première variante de l'invention, le composé hydrosoluble ou hydrodispersable, est un polymère issu de la polymérisation :
□ d'au moins un monomère (I) acide monocarboxylique ou polycarboxylique, ou anhydride, aliphatique, cyclique ou aromatique, linéaire ou ramifié, éthyléniquement insaturé, et
□ d'au moins un monomère hydrocarboné (II), linéaire ou ramifié, monoéthyléniquement ou polyéthyléniquement insaturé, et/ou
□ d'au moins un monomère (III) ester polyalcoxylé d'acide carboxylique éthyléniquement insaturé.

Selon un mode de réalisation particulier de cette première variante, on peut citer tout d'abord les polymères issus de la polymérisation :
□ d'au moins un monomère de formule (I) :

   (R¹)(R¹) - C = C(R'¹) - COOH (I)

   formule dans laquelle les radicaux R¹, R'¹, identiques ou différents, représentent un atome d'hydrogène, un radical hydrocarboné en C₁ - C₁₀ comprenant éventuellement un groupement - COOH, un groupement - COOH ; et
□ d'au moins un monomère de formule (II) :

   (R2)(R2) - C = CH₂ (II)

   formule dans laquelle les radicaux R², identiques ou différents, représentent un radical hydrocarboné, linéaire ou ramifié, en C₁ - C₁₀. Plus particulièrement, lesdits radicaux sont des radicaux alkyles ou alcényle, ces derniers pouvant comprendre une ou plusieurs insaturations éthyléniques. De préférence, lesdits radicaux ne comprennent pas d'hétéroatomes.

Selon un mode de réalisation préféré de l'invention le monomère de formule (I) est tel que l'un des radicaux R¹ est un atome d'hydrogène ; l'autre radical R¹ représente un atome d'hydrogène, un groupement -COOH ou un groupement - (CH₂)ₙ - COOH dans lequel n est compris entre 1 et 4, un radical alkyle en C₁-C₄ ; R'¹ représente un atome d'hydrogène, en groupement -(CH₂)ₘ - COOH dans lequel m est compris entre 1 et 4, un radical alkyle en C₁-C₄.

De préférence, l'une des radicaux R¹ représente un atome d'hydrogène, l'autre radical R¹ représente un atome d'hydrogène, un groupement -COOH ou (CH₂)-COOH, un radical méthyle, et R'¹, représente un atome d'hydrogène, un groupement -CH₂ COOH ou un radical méthyle.

Selon un mode de réalisation plus particulier, le monomère de formule (I) est choisi parmi les acides ou anhydrides acrylique, méthacrylique, citraconique maléique, fumarique, itaconique, crotonique.

En ce qui concerne le monomère de formule (II), ce dernier peut être notamment choisi parmi l'éthylène, le propylène, le 1-butène, l'isobutylène, le n-1-pentène, le 2-méthyl 1-butène, le n-1-hexène, le 2-méthyl 1-pentène, le 4-méthyl 1-pentène, le 2-éthyl 1-butène, le diisobutylène (ou 2,4,4-triméthyl 1-pentène), le 2-méthyl 3,3-diméthyl 1-pentène.

Selon un mode de réalisation avantageux de l'invention, le rapport molaire de monomère (I) / monomère (II) est compris entre 30/70 et 70/30.

De préférence, le copolymère de formule (i) est issu de la polymérisation de l'anhydride maléique et du 2,4,4 - triméthyl 1-pentène.

Il est précisé que le polymère (i) est obtenu plus particulièrement en effectuant une polymérisation radicalaire des monomères (I) et (II).

Ces composés sont bien connus de l'homme de l'art. A titre de polymère de ce type, on peut citer celui commercialisé sous la dénomination Geropon^{®} T36 (anhydride maléique/diisobutylène), commercialisé par Rhodia Chimie, ainsi que le Sokalan^{®} CP9 (anhydride maléique/oléfine) commercialisé par BASF.

Un second mode de réalisation particulier de cette première variante consiste à mettre en oeuvre un polymère (i) obtenu par polymérisation d'au moins un monomère de formule (I) décrit précédemment avec au moins un monomère de formule (III) :

CH₂=C(R³)-C(O)-O-[CH₂CH(R⁴)O]ₘ -[CH(R⁵)-CH₂O]ₙ - R⁶

formule dans laquelle :
R3 est un atome d'hydrogène ou un radical méthyle,
R4 et R⁵ identiques ou différents, représentent un atome d'hydrogène ou un radical alkyle contenant de 1 à 4 atomes de carbone,
R⁶ est un radical alkyle, aryle, alkylaryle ou arylalkyle contenant de 1 à 30, de préférence de 8 à 30 atomes de carbone,
n est compris entre 2 et 100, de préférence entre 6 et 100 et m est compris entre 0 et 50, sous réserve que n soit supérieur ou égal à m et leur somme comprise entre 2 et 100, de préférence entre 6 et 100.

De préférence, on utilise des monomères de formule (III) pour lesquels R⁶ est un radical alkyle contenant de 8 à 30 atomes de carbone, un radical phényle substitué par un à trois groupements 1-phényléthyles, un radical alkylphényle dont le radical alkyle content de 8 à 16 atomes de carbone.

Parmi les monomères de ce type, susceptibles d'être mis en oeuvre, on peut citer ceux décrits dans les brevets EP 705854, US 4 138 381 ou encore US 4 384 096.

Le polymère obtenu par réaction des monomères (I) et (III) est de préférence obtenu par polymérisation radicalaire.

Notons que l'on ne sortirait pas du cadre de la présente invention en utilisant un polymère (i) comprenant les trois monomères qui viennent d'être décrits.

A titre d'indication, la masse moléculaire en poids des polymères (i) est plus particulièrement inférieure à 20000 g/mole ; masses moléculaires absolues, en poids, déterminées par chromatographie par exclusion stérique couplée à la méthode MALLS.

Il est précisé que la masse moléculaire du polymère ainsi que les proportions respectives des monomères (I) et (II) et/ou (III) sont telles que le polymère résultant soit hydrosoluble ou hydrodispersable au sens de la présente invention.

Une deuxième variante de la présente invention est constituée par l'utilisation, en tant que composé hydrosoluble ou hydrodispersable, d'au moins un polymère issu de la polymérisation d'au moins un monomère de formule (I) telle que définie précédemment et comportant, en outre, éventuellement au moins un greffon hydrophobe hydrocarboné en C₄ - C₃₀, saturé ou non, aromatique ou non, éventuellement interrompu par un ou plusieurs hétéroatomes.

Tout ce qui a été indiqué auparavant à propos du monomère de formule (I), formules générales et préférées, reste applicable dans le cas du polymère (ii) et ne sera donc pas repris ici.

Notons que ce polymère est obtenu de préférence, en mettant oeuvre une polymérisation radicalaire.

Par ailleurs, le greffon hydrophobe est choisi parmi les radicaux aliphatiques, cycliques, aromatiques, alkylaromatiques, arylaliphatiques comprenant 4 à 30 atomes de carbone, et pouvant être interrompus par un ou plusieurs hétéroatomes, de préférence l'oxygène.

Il est à noter que les greffons sont reliés au squelette du polymère par l'intermédiaire de groupements ester et/ou amide.

De tels polymères greffés sont obtenus en mettant en oeuvre des méthodes connues de l'homme de l'art consistant dans un premier temps à faire polymériser, de préférence par voie radicalaire le ou les monomères (I), puis à faire réagir une partie des fonctions carboxyliques libres, avec des réactifs choisis notamment parmi les alcools ou les amines hexylique, heptylique, laurylique, béhénique, éventuellement éthoxylés et/ou propoxylés, les mono-, di- ou tristyrylphénols éventuellement éthoxylés et/ou propoxylés.

A titre indicatif, la masse moléculaire en poids des polymères (ii) est plus particulièrement inférieure à 20000 g/mole ; masses moléculaires absolues, en poids, déterminées par chromatographie par exclusion stérique couplée à la méthode MALLS.

Cependant, la masse moléculaire ainsi que les proportions respectives de monomère(s) (I) et de greffons hydrophobes, s'ils sont présents, est telle que le polymère résultant soit hydrosoluble ou hydrodispersable au sens de la présente invention.

Les polymères (i) et (ii) peuvent en outre comprendre des motifs correspondant à des monomères non-ioniques monoéthyléniquement insaturés (IV) autres que les monomères (II).

Parmi les monomères non-ioniques monoéthyléniquement insaturés (IV), on peut citer :
□ les monomères vinylaromatiques comme le styrène, le vinyltoluène,
□ les C₁-C₂₀ alkylesters d'acides α-β éthyléniquement insaturés comme les acrylates ou méthacrylates de méthyle, éthyle, butyle,
□ les esters de vinyle ou d'allyle d'acides α-β éthyléniquement insaturés comme les acétates ou propionates de vinyle ou d'allyle,
□ les halogénures de vinyle ou de vinylidène comme le chlorure de vinyle ou de vinylidène,
□ les nitriles α-β éthyléniquement insaturés comme l'acrylonitrile,
□ les hydroxyalkylesters d'acides α-β éthyléniquement insaturés comme les acrylates ou méthacrylates d'hydroxyéthyle ou d'hydroxypropyle,
□ les amides α-β éthyléniquement insaturés comme l'acrylamide, le méthacrylamide.

Si de tels monomères sont présents, la polymérisation a lieu en leur présence.

Une troisième variante de la présente invention, consiste à employer en tant que composé hydrosoluble ou hydrodispersable, au moins une prptéine, ou au moins un polypeptide d'origine naturelle ou synthétique, comportant éventuellement au moins un greffon hydrophobe, hydrocarboné, en C₄-C₃₀ saturé ou non, aromatique ou non, éventuellement interrompu par un ou plusieurs hétéroatomes.

Les polymères peptidiques d'origine naturelle ou synthétique sont des polymères dérivés de la polycondensation d'acides aminés, notamment de l'acide aspartique et glutamique ou des précurseurs des diaminodiacides, et hydrolyse. Ces polymères peuvent être aussi bien des polymères dérivés de l'acide aspartique ou glutamique, que des polymères dérivés de l'acide aspartique et de l'acide glutamique en proportions quelconques, ou des copolymères dérivés de l'acide aspartique et/ou glutamique et d'autres aminoacides. Parmi les aminoacides polymérisables, on peut entre autres, citer la glycine, l'alanine, la leucine, l'isoleucine, la phényl alanine, la méthionine, l'histidine, la proline, la lysine, la sérine, la thréonine, la cystéine...

Parmi les polypeptides d'origine naturelle, on peut mentionner les protéines d'origine végétale ou animale, hydrosolubles ou hydrodispersables. Les protéines d'origine végétales sont de préférence des hydrolysats de protéines. Leur degré d'hydrolyse est plus particulièrement inférieur ou égal à 40 %.

Parmi les protéines d'origine végétale, on peut citer à titre indicatif, les protéines provenant des graines protéagineuses, notamment celles de pois, féverole, lupin, haricot et lentille ; les protéines provenant de grains de céréales notamment celles de blé, orge, seigle, maïs, riz, avoine, millet ; les protéines provenant de graines oléagineuses notamment celles du soja, arachide, tournesol, colza et noix de coco ; les protéines provenant de feuilles notamment luzerne et ortie ; les protéines provenant d'organes végétaux et réserves enterrées notamment celles de pomme de terre, betterave.

Parmi les protéines d'origine animale, on peut citer par exemple, les protéines musculaires, notamment les protéines du stroma, la gélatine ; les protéines provenant du lait, notamment caséine, lactoglobuline ; et les protéines de poissons.

Les protéines d'origine végétale et plus particulièrement les protéines provenant du soja et du blé sont préférées.

Ce qui a été indiqué auparavant à propos de la nature du greffon hydrophobe reste valable et ne sera pas repris ici.

Il est à noter que les greffons hydrophobes peuvent être liés au polypeptide par l'intermédiaire de liaisons amide, ester, urée, uréthanne, isocyanate, amino.

Les polymères greffés sont obtenus par réaction d'une partie des fonctions amines ou acides libres, avec des composés permettant de créer les liaisons précitées.

Les composés préférés présentent un degré de polymérisation faible. Plus particulièrement, à titre d'illustration, la masse moléculaire en poids est inférieure à 20000 g/mole ; masses moléculaires absolues, en poids, déterminées par chromatographie par exclusion stérique couplée à la méthode MALLS.

Il est précisé que la masse moléculaire du polymère, ainsi que la proportion de greffon par rapport au polypeptide, lorsqu'il est présent, sont telles que le polymère résultant soit hydrosoluble ou hydrodispersable au sens de la présente invention.

Selon une quatrième variante de la présente invention, le composé hydrosoluble ou hydrodispersable est choisi parmi les polysaccharides comportant éventuellement un greffon hydrophobe hydrocarboné, en C₄ - C₃₀, saturé ou non, aromatique ou non, éventuellement interrompu par un ou plusieurs hétéroatomes.

A titre d'exemple non limitatif de polysaccharides convenables, on peut citer entre autres l'amidon, l'amidon modifié, les alginates, les hydoxyalkylcelluloses ou leurs dérivés.

Selon un mode de réalisation préféré de l'invention, le composé hydrosoluble ou hydrodispersable est choisi parmi les polysaccharides fortement dépolymérisés comportant éventuellement un greffon hydrophobe hydrocarboné, en C₄ - C₃₀, saturé ou non, aromatique ou non, éventuellement interrompu par un ou plusieurs hétéroatomes.

De tels composés sont notamment décrits dans l'ouvrage de P. ARNAUD intitulé "cours de chimie organique", GAUTHIER-VILLARS éditeurs, 1987.

Parmi de tels composés, on peut citer ceux obtenus à partir du dextran, d'amidon, de maltodextrine, de gomme xanthane et de galactomannanes tels que le guar ou la caroube.

Ces polysaccharides fortement dépolymérisés présentent de préférence un point de fusion supérieur à 100°C et une solubilité dans l'eau comprise entre 50 et 500 g/l, à 25°C.

Au sujet des greffons hydrophobes, on pourra se référer à ce qui a été indiqué auparavant.

Il est à noter que les greffons hydrophobes peuvent être liés au polysaccharide par l'intermédiaire de liaisons ester, amide, urée, uréthanne, isocyanate, amino.

Les polymères greffés sont obtenus par réaction d'une partie des fonctions alcools ou acides libres, avec des composés permettant de créer les liaisons précitées.

Plus particulièrement, à titre d'illustration, la masse moléculaire en poids de ces polysaccharides fortement dépolymérisés est inférieure à 20000 g/mole ; masses moléculaires absolues, en poids, déterminées par chromatographie par exclusion stérique couplée à la méthode MALLS.

Cependant, il est précisé que la masse moléculaire du polysaccharide, qu'il soit ou non fortement dépolymérisé, ainsi que la proportion de greffon s'il est présent, par rapport audit polysaccharide, sont telles que le polymère résultant soit hydrosoluble ou hydrodispersable au sens de la présente invention.

Selon une dernière variante de l'invention, le composé hydrosoluble ou hydrodispersable est un alcool polyvinylique, de la polyvyinylpyrrolidone.

Il est à noter que l'alcool polyvinylique peut être partiellement ou totalement sous forme hydrolysée, dès l'instant qu'il est hydrosoluble ou hydrodispersable au sens de l'invention.

Bien entendu, il est tout à fait envisageable d'utiliser ces différents types de composés hydrosolubles ou hydrodispersables en combinaison.

La teneur en composé hydrosoluble ou hydrodispersable dans la phase aqueuse externe est par ailleurs telle que la teneur en ce composé dans le granulé final est plus particulièrement comprise entre 30 et 85 % en poids par rapport au poids dudit granulé.

Il est à noter que dans le cas plus particulier où le composé hydrosoluble ou hydrodispersable est choisi parmi les protéines, l'alcool polyvinylique, la polyvinylpyrrolidone ou parmi les polysaccharides, la teneur en ce composé dans le granulé final est plus particulièrement comprise entre 50 et 85 % en poids par rapport au poids dudit granulé.

En outre, dans le cas où le composé hydrosoluble ou hydrodispersable est choisi parmi les variantes (i), (ii), les polypeptides et les polysaccharides fortement dépolymérisés, la teneur en ce composé dans le granulé final est de manière très avantageuse comprise entre 30 et 70 % en poids par rapport au poids dudit granulé.

De plus, le rapport pondéral émulsion inverse / phase aqueuse externe est habituellement compris entre 30/70 et 90/10, de préférence comprise entre 50/50 et 90/10.

Afin d'équilibrer les pressions osmotiques de la phase aqueuse externe et de la phase aqueuse interne, on peut ajouter dans la phase aqueuse externe au moins un additif choisi parmi les sels tels que les halogénures de métaux alcalins ou alcalino-terreux (comme le chlorure de sodium, le chlorure de calcium), au moins un sulfate de métal alcalin ou alcalino-terreux (comme le sulfate de calcium) ; ou choisi parmi les sucres (glucose par exemple), ou parmi les polysaccharides (notamment le dextran) ou leurs mélanges.

Les concentrations en additif (sel, en sucre et/ou en polysaccharide) sont telles que les pressions osmotiques des phases aqueuses externe et interne sont équilibrées.

De plus, en fonction de l'application à laquelle est destinée le granulé selon l'invention, ou en fonction de la nature de la matière active, il peut être avantageux d'ajuster le pH de la phase aqueuse externe par ajout de base (soude, potasse) ou d'acide (chlorhydrique), une fois l'émulsion multiple obtenue.

A titre illustratif, la gamme de pH habituelle de la phase aqueuse externe est comprise entre 3 et 8, de préférence entre 5 et 8.

Selon une variante avantageuse de la présente invention, la phase aqueuse de l'émulsion peut comprendre au moins un polymère épaississant. Ce polymère a pour effet d'éviter le crémage et/ou la sédimentation de l'émulsion finale.

A titre d'illustration, on peut utiliser des polymères épaississants extraits de végétaux et éventuellement modifiés, tels que les carraghénannes, les alginates, les carboxyméthyl celluloses, les méthylcelluloses, les hydroxypropyl celluloses, les hydroxyéthyl celluloses, les gellanes.

On peut de même employer des polymères épaississants du type des polysaccharides d'origine animale, végétale, bactérienne, on peut citer à titre d'exemple non limitatif, la gomme xanthane, le guar et dérivés (tels que l'hydroxypropyl guar par exemple), les polydextroses, ou leurs combinaisons.

Lorsqu'il est présent, la teneur en polymère épaississant est plus particulièrement comprise entre 0,1 et 2 % en poids par rapport à la phase aqueuse externe, de préférence entre 0,1 et 0,5 % en poids par rapport à la phase aqueuse externe. Précisons que dans cette gamme de concentration, le polymère épaississant est soluble dans la phase aqueuse.

On ne sortirait pas du cadre de la présente invention en mélangeant plusieurs émulsions multiples, dès l'instant que les phases aqueuses externes des émulsions mélangées sont compatibles.

Selon une variante de la présente invention, la phase aqueuse externe peut comprendre une phase organique externe dispersée et/ou un solide dispersé.

Tout ce qui a été indiqué précédemment concernant la matière active hydrophobe éventuellement présente dans la phase organique interne, reste valable et ne sera pas détaillé à nouveau maintenant.

Notons que ces matières actives peuvent être mises en oeuvre en présence d'additifs classiques dans le domaine d'application concerné.

Au cas où une telle phase organique externe est présente, elle représente plus particulièrement 1 à 50 % en poids de la phase aqueuse externe, de préférence 5 à 25 % en poids de la phase aqueuse externe.

En outre, il est préférable que la taille des gouttelettes de la phase organique externe soit au plus du même ordre de grandeur que celle de l'émulsion inverse dispersée dans la phase aqueuse externe.

Quant à la possibilité de mettre en oeuvre un solide dispersé dans la phase aqueuse externe, tous les solides utilisés dans les diverses applications mentionnées peuvent convenir. De préférence, la taille de ce solide dispersé est voisine ou plus faible que celle des gouttelettes de l'émulsion inverse.

Au cas où le solide dispersé est présent, sa teneur représente plus particulièrement 1 à 50 % en poids de la phase aqueuse externe, de préférence 5 à 25 % en poids.

La préparation de l'émulsion multiple peut être réalisée selon toute méthode connue.

A titre d'exemple de préparation de l'émulsion multiple, dans un cas où l'on n'utilise pas de polymère thermoépaississant, on peut procéder en mélangeant l'eau, le tensioactif non ionique et/ou le polymère amphiphile non ionique, et éventuellement l'additif. De préférence, on mélange en premier lieu l'eau et le tensioactif et/ou le polymère amphiphile, sous agitation ; puis on ajoute le composé hydrosoluble ou hydrodispersable.

Cette opération a lieu habituellement à une température supérieure à la température de fusion du composé utilisé en tant que phase organique interne. De préférence, cette température est comprise entre 20 et 80°C.

La phase aqueuse externe peut éventuellement être laissée au repos pendant 1 à 12 heures à température ambiante.

On procède ensuite à la préparation de l'émulsion multiple proprement dite en ajoutant l'émulsion inverse à la phase aqueuse externe.

Cette opération a lieu sous agitation, en ajoutant au départ l'émulsion inverse lentement.

L'agitation peut être faite au moyen d'une pale cadre. Typiquement, la vitesse d'agitation est relativement lente, de l'ordre de 400 tr/minute.

Dans le cas où l'on utilise un polymère thermoépaississant, plusieurs variantes sont envisageables.

Par exemple, la phase aqueuse externe est préparée en mélangeant ses divers éléments constitutifs. Plus particulièrement on mélange l'eau, le tensioactif et/ou polymère amphiphile. On porte ensuite la température du mélange à une température supérieure ou égale à la température d'épaississement du polymère thermoépaississant. Il est à noter que la température est avantageusement supérieure ou égale à la température de fusion du composé utilisé en tant que phase organique interne.

Puis on ajoute à la phase aqueuse externe ainsi obtenue, l'émulsion inverse. Cette opération a lieu à une température supérieure ou égale à la température d'épaississement du polymère thermoépaississant.

Une fois l'émulsion multiple obtenue, on porte l'ensemble à une température inférieure à la température d'épaississement du polymère thermoépaississant et l'on ajoute ensuite le composé hydrosoluble ou hydrodispersable, sous agitation.

Les conditions d'agitation sont du même type que la variante précédente, à savoir une agitation lente, de l'ordre de 400 tours/ minute.

Selon une autre possibilité, l'ensemble de tous les éléments constitutifs de la phase aqueuse externe sont mélangés. De préférence, on ajoute à l'eau, le tensioactif et/ou polymère amphiphile, éventuellement l'additif, le polymère thermoépaississant puis le composé hydrosoluble ou hydrodispersable, sous agitation.

Puis on ajuste la température du mélange de manière à ce qu'elle soit supérieure ou égale à la température d'épaississement du polymère thermoépaississant et/ou à la température de fusion du composé utilisé comme phase organique de l'émulsion inverse. On ajoute ensuite l'émulsion inverse dans la phase aqueuse externe à la température adéquate.

L'agitation est de préférence lente, de l'ordre de 400 à 700 tours/minute.

Dans le cas où la phase aqueuse externe comprend une phase organique externe dispersée, l'émulsion multiple est obtenue de préférence en dispersant l'émulsion inverse dans la phase aqueuse externe. On ajoute ensuite l'émulsion directe composée de la phase organique externe dispersée dans la même phase aqueuse externe. Bien évidemment, les quantités de phase aqueuse externe introduites avec les émulsions inverse et directe sont telles que les proportions pondérales de chacune des phases satisfont aux conditions explicitées ci-dessus pour l'émulsion multiple.

L'obtention de l'émulsion directe est réalisée selon toute méthode connue, par mélange sous agitation des deux phases : la phase organique externe comprenant la matière active hydrophobe et la phase aqueuse externe comprenant le tensioactif et/ou polymère amphiphile, le composé hydrosoluble ou hydrodispersable et éventuellement le polymère thermoépaississant.

Dans le cas où la phase aqueuse externe comprend un solide dispersé, l'obtention de l'émulsion multiple peut être réalisée comme indiqué dans le premier cas, puis on ajoute ledit solide dispersé dans la phase aqueuse externe.

La taille moyenne des gouttelettes de l'émulsion multiple varie avantageusement entre 5 et 100 µm, plus particulièrement entre 5 et 50 µm, avantageusement entre 5 et 15 µm. Elles sont mesurées au moyen d'un granulomètre Horiba, et correspondent au diamètre médian en volume (d50) qui représente le diamètre de la particule égal à 50 % de la distribution cumulative.

L'émulsion multiple est ensuite séchée de manière à obtenir les granulés selon l'invention.

L'opération de séchage peut être effectuée par tout moyen connu de l'homme du métier.

L'opération de séchage est conduite dans des conditions telles que la phase aqueuse externe est éliminée.

De préférence, le séchage est effectué de telle sorte que qu'au moins 90% en poids de la phase aqueuse externe sont éliminés, de préférence entre 90 et 95 % en poids.

En effectuant le séchage dans de telles conditions, les granulés séchés selon la présente invention comprennent une teneur en eau interne comprise entre 10 et 50 % en poids du granulé, de préférence entre 20 et 30 % en poids du granulé.

Ainsi, selon un premier mode de réalisation de l'invention, on peut envisager un séchage en étuve. De préférence, ce séchage a lieu en couche mince.

Habituellement, la température de séchage est inférieure ou égale à 100°C. Plus particulièrement, des températures comprises entre 50 et 90°C conviennent à la mise en oeuvre de cette méthode.

Selon un autre mode de réalisation préféré de l'invention, on utilise une méthode de séchage dite rapide, de l'émulsion multiple.

Conviennent à ce titre le séchage par atomisation, ou mettant en oeuvre des tambours Duprat^{®}, ou encore la lyophilisation (congélation-sublimation).

Ces modes de séchage, comme notamment le séchage par atomisation, sont particulièrement indiqués car ils permettent de conserver l'émulsion multiple en l'état et d'obtenir directement des granulés.

Le séchage par atomisation peut s'effectuer de manière habituelle dans tout appareil connu tel que par exemple une tour d'atomisation associant une pulvérisation réalisée par une buse ou une turbine avec un courant de gaz chaud.

La température de sortie des gaz d'atomisation est de préférence comprise entre 55 et 75°C. Ces températures sont données à titre indicatif, et dépendent de la stabilité thermique des divers éléments. De plus, elle est définie selon la teneur en eau finale souhaitée dans le granulé.

Dans le cas d'opérations de séchage de l'émulsion multiple réalisées au moyen de tambour Duprat^{®}, ou de tout moyen permettant d'obtenir rapidement un film sec qui est séparé du support séchant par une opération de raclage par exemple, on obtient des granulés que l'on peut éventuellement broyer. Si nécessaire, ces granulés peuvent faire l'objet d'une mise en oeuvre ultérieure, comme une étape d'agglomération, de manière à obtenir des granulés agglomérés.

Il est à noter que des additifs, tels que les agents anti-mottants peuvent être incorporés aux granulés au moment de cette seconde étape de séchage.

Il est de même possible, à titre d'exemple, d'utiliser une charge choisie notamment parmi le carbonate de calcium, le sulfate de baryum, le kaolin, la silice, la bentonite, l'oxyde de titane, le talc, l'alumine hydràtée et le sulfoaluminate de calcium.

Les additifs peuvent être introduits avant le séchage de l'émulsion multiple, comme cela peut notamment être le cas des charges. Ils peuvent aussi être co-séchés avec l'émulsion multiple, comme c'est notamment le cas des anti-mottants.

De manière avantageuse, la taille moyenne des granulés obtenus directement après séchage est comprise entre 100 µm et quelques millimètres, de préférence entre 100 et 800 µm. La taille moyenne des granulés est mesurée avec un appareil Sympatec et correspond au diamètre médian en volume (d50) qui représente le diamètre de la particule égal à 50 % de la distribution cumulative.

Les granulés selon l'invention, lorsqu'ils sont dispersés dans une phase aqueuse permettent d'obtenir à nouveau une émulsion multiple.

Les granulés selon l'invention peuvent être stockés et transportés sans aucune difficulté.

Un exemple concret mais non limitatif de l'invention va maintenant être présenté.

### EXEMPLE

### 1/ Emulsion inverse

Composition de l'émulsion inverse :
* 30% de phase aqueuse :
   - 14% d'acide lactique (% en poids de solution à 0,1 M exprimé par rapport au poids de phase aqueuse)
   - 86% de NaCl (% en poids de solution à 0,1M exprimé par rapport au poids de phase aqueuse)
* 70% de phase huile (huile de soja et agent tensioactif) comprenant 5% d'agent tensioactif (Arlacel P135 ; ICI - Uniquema (*); % exprimé en poids par rapport au poids de phase aqueuse).
(*) Arlacel P 135 : Polyhydroxystéarate - PEG - Polyhydroxystéarate ; HLB = 5-6 ; Mw≈ 5000 g/mol)

### Préparation de l'émulsion inverse

On prépare 300 g d'émulsion inverse, comprenant 210 g de phase organique interne et 90 g de phase aqueuse interne.

D'une part, on mélange 12,6 g de solution d'acide lactique à 0,1 M avec 77,4 g de solution de NaCl à 0,1M.

D'autre part, on mélange 4,5 g d'Arlacel P135 avec 205,5 g d'huile de soja. Préalablement au mélange, l'huile de soja et l'Arlacel ont été placés à l'étuve à 75°C.

On ajoute ensuite la phase aqueuse interne dans la phase organique interne, sous agitation à l'Ultraturrax à 9500 tr/min. Le mélange est effectué à 75 °C. On effectue ensuite un affinage à 9500 tr/min pendant 8 minutes.

### 2/ Emulsion multiple

### Composition de l'émulsion multiple :

* 65% d'émulsion inverse
* 35% d'extrait sec en phase aqueuse externe contenant :
   - 2% d'Arlatone F127G (**) (ICI - Uniquema ; % en poids exprimé par rapport au poids de l'émulsion inverse) ;
   - 51,8% de Géropon T36 (***) (Rhodia Chimie; % en poids de la solution de Geropon T 36; exprimé par rapport au poids de l'émulsion inverse) ;
   - 47,2% de solution d'acide chlorhydrique à 2N (% en poids exprimé par rapport au poids de Géropon T36).
(**) Arlatone F127G : HO(CH₂CH₂O)ₓ(OCH(CH₃)CH₂O)_{y}(CH₂CH₂O)_{z}H avec vérification de l'inéquation suivante : 82<x+z<90 et le polymère comprend 7 motifs OP pour 1 mole de produit).
(***) Geropon T36 : Copolymère anhydride maléique / diisobutylène ; Mw = 5000 g/mol.

### Préparation de l'émulsion multiple

### Préparation de la phase aqueuse externe :

Dans un bécher de 2 litres, on dissout 6 g d'Arlatone F127G dans 200 g d'eau, sous agitation à la pale cadre à 300 tr/min pendant 30 minutes.

On ajoute ensuite à la solution d'Arlatone F127G, 622,15 g de solution de Géropon T36 (solution dans l'eau à 25 % en poids).

### Préparation de l'émulsion multiple :

On introduit les 300 g de l'émulsion inverse obtenue au point 1/, sous agitation à la pale cadre à 300 tr/min, goutte à goutte puis doucement, dans la phase aqueuse externe, à température ambiante.

Immédiatement après l'introduction de l'émulsion inverse, on ajoute 73,8 g de solution de HCl (2N), en une seule fois, sous agitation à 400 tr/min, à température ambiante.

On maintient l'agitation pendant 1 heure à 400 tr/min.

Le pH de la phase aqueuse externe de l'émulsion multiple ainsi obtenue est de 7,5.

La taille moyenne des gouttelettes de l'émulsion multiple est d'environ de 10 à 15 µm (granulomètre Horiba).

### 3/ Séchage de l'émulsion multiple

On effectue une atomisation de l'émulsion multiple.

Les gouttes d'émulsion multiple produites traversent un courant d'air chaud tangentiel descendant et forment en séchant des granulés qui sont recueillis en partie basse.

Les conditions d'atomisation sont les suivantes :
T entrée : 110°C / T sortie : 60°C
P de pulvérisation sur une buse bifluide : 2 bar absolus
V émulsion multiple séchée : 600 ml/M émulsion sèche : ≈ 132 g

### 4/ Test de redispersion des granulés

A l'aide d'une spatule, quelques milligrammes d'émulsion sèche sont redispersés dans de l'eau distillée.

La redispersion est immédiate et l'on obtient à nouveau une émulsion multiple dont la taille des gouttelettes est comprise entre 10 et 15 µm (selon les observations en microscopie optique et au granulomètre Horiba).

## Revendications

1. Granulés susceptibles d'être obtenus par séchage d'une émulsion inverse, dispersée dans une phase aqueuse externe, ledit séchage éliminant la phase aqueuse externe.
(a) l'émulsion inverse comprenant une phase aqueuse interne comprenant au moins une matière active hydrophile, dispersée dans une phase organique interne, ladite émulsion inverse comprenant au moins un tensioactif non ionique et/ou au moins un polymère amphiphile à blocs, et/ou au moins un tensioactif cationique ;
(b) la phase aqueuse externe comprenant :
- au moins un tensioactif polyalcoxylé non ionique et/ou au moins un polymère amphiphile polyalcoxylé non ionique,
- au moins un polymère hydrosoluble ou hydrodispersable se présentant sous une forme solide en présence d'une teneur en eau d'au plus 10 % en poids par rapport au poids dudit polymère et dont la température de transition vitreuse est supérieure à 25°C, de préférence supérieure à 50°C.

2. Granulés selon la revendication 1, **caractérisés en ce que** le tensioactif non ionique et le polymère amphiphile à blocs, sont choisis parmi ceux qui vérifient à la fois les deux conditions ci-dessous :
- lorsqu'ils sont mélangés avec la phase organique interne, à une concentration comprise entre 0,1 et 10 % en poids de ladite phase à 25°C, se trouvent sous la forme d'une solution dans tout ou partie de la gamme de concentration indiquée ;
- lorsqu'ils sont mélangés avec la phase aqueuse interne, à une concentration comprise entre 0,1 et 10 % en poids de ladite phase et à 25°C, se trouvent sous la forme d'une dispersion dans tout ou partie de la gamme de concentration indiquée.

3. Granulés selon l'une des revendications précédentes, **caractérisés en ce que** la quantité totale de tensioactif non ionique, de polymère amphiphile à blocs et/ou de tensioactif cationique représente 2 à 10 % en poids de la phase aqueuse interne.

4. Granulés selon l'une des revendications précédentes, **caractérisés en ce que** l'émulsion inverse présente une proportion pondérale phase aqueuse / phase organique comprise entre 10/90 et 90/10, de préférence, entre 30/70 et 80/20.

5. Granulés selon l'une des revendications précédentes, **caractérisés en ce que** la phase aqueuse interne comprend au moins un additif choisi parmi les sels tels que les halogénures de métaux alcalins ou alcalino-terreux, ou les sulfates de métal alcalin ou alcalino-terreux, parmi les sucres, ou parmi les polysaccharides, ou leurs mélanges.

6. Granulés selon la revendication précédente, **caractérisés en ce que** la concentration en sel dans la phase aqueuse interne est comprise entre 0,05 et 1 mol/l, de préférence 0,1 à 0,4 mol/l ; la concentration en sucre et/ou polysaccharide est telle que la pression osmotique de la phase aqueuse interne comprenant le sucre et/ou le polysaccharide correspond à la pression osmotique d'une phase aqueuse interne comprenant 0,05 à 1 mol/l de sel.

7. Granulés selon l'une des revendications précédentes, **caractérisés en ce que** la phase organique interne comprend au moins une matière active hydrophobe, compatible avec la matière active hydrophile présente dans la phase aqueuse interne.

8. Granulés selon l'une des revendications précédentes, **caractérisés en ce que** le tensioactif et le polymère présents dans la phase aqueuse externe, sont choisis parmi ceux qui vérifient à la fois les deux conditions ci-dessous :
- lorsqu'ils sont mélangés avec la phase aqueuse externe, à une concentration comprise entre 0,1 et 10 % en poids de ladite phase à 25°C, se trouvent sous la forme d'une solution dans tout ou partie de la gamme de concentration indiquée ;
- lorsqu'ils sont mélangés avec la phase organique interne, à une concentration comprise entre 0,1 et 10 % en poids de ladite phase et à 25°C, se trouvent sous la forme d'une dispersion dans tout ou partie de la gamme de concentration indiquée.

9. Granulés selon l'une des revendications précédentes, **caractérisés en ce que** la phase aqueuse externe comprend au moins un polymère thermoépaississant.

10. Granulés selon l'une des revendications précédentes, **caractérisés en ce que** le polymère thermoépaississant est choisi parmi les polymères présentant un saut de viscosité entre 25 et 80°C tel que la valeur du rapport log₁₀ (viscosité à 80°C) /log₁₀ (viscosité à 25°C) est au moins égale à 1, de préférence au moins égale à 2, et parmi ceux dont la variation de viscosité est réversible.

11. Granulés selon l'une des revendications précédentes 9 ou 10, **caractérisés en ce que** la teneur en polymère thermoépaississant est comprise entre 0,2 et 10 % en poids de la phase aqueuse externe, de préférence, entre 1 et 5 % en poids de la phase aqueuse externe.

12. Granulés selon la revendication précédente, **caractérisés en ce que** le polymère hydrosoluble ou hydrodispersable est choisi parmi :
(i) au moins un polymère obtenu par polymérisation
□ d'au moins un monomère (I) acide monocarboxylique ou polycarboxylique, ou anhydride, aliphatique, cyclique ou aromatique, linéaire ou ramifié, éthyléniquement insaturé, et
□ d'au moins un monomère hydrocarboné, linéaire ou ramifié, monoéthyléniquement ou polyéthyléniquement insaturé, et/ou
□ d'au moins un monomère (III) ester polyalcoxylé d'acide carboxylique éthyléniquement insaturé ;
(ii) au moins un polymère issu de la polymérisation d'au moins un monomère (I) acide monocarboxylique ou polycarboxylique, ou anhydride, aliphatique, cyclique ou aromatique, linéaire ou ramifié, éthyléniquement insaturé, et comprenant éventuellement au moins un greffon hydrophobe, hydrocarboné, en C₄-C₃₀, saturé ou non, aromatique ou non, éventuellement interrompu par un ou plusieurs hétéroatomes :
(iii) les protéines, les polypeptides d'origine naturelle ou synthétique, comportant éventuellement au moins un greffon hydrophobe, hydrocarboné, en C₄-C₃₀, saturé ou non, aromatique ou non, éventuellement interrompu par un ou plusieurs hétéroatomes ;
(iv) les polysaccharides, de préférence fortement dépolymérisés comportant éventuellement au moins un greffon hydrophobe, hydrocarboné, en C₄-C₃₀, saturé ou non aromatique ou non, éventuellement interrompu par un ou plusieurs hétéroatomes ;
(v) l'alcool polyvinylique, la polyvinylpyrrolidone.

13. Granulés selon l'une des revendications précédentes, **caractérisés en ce que** le composé hydrosoluble ou hydrodispersable présent dans la phase aqueuse externe est un polymère (i) issu de la polymérisation
□ d'au moins un monomère de formule (I) :
(R¹)(R¹) - C = C(R'¹) - COOH (I)
formule dans laquelle les radicaux R¹, R'¹, identiques ou différents, représentent un atome d'hydrogène, un radical hydrocarboné en C₁ - C₁₀ comprenant éventuellement un groupement - COOH, un groupement - COOH ; et
□ d'au moins un monomère de formule (II) :
(R²)(R²) - C = CH₂ (II)
formule dans laquelle les radicaux R², identiques ou différents, représentent un radical hydrocarboné, linéaire ou ramifié, en C₁ - C₁₀ ; et/ou
□ d'au moins un monomère de formule (III) :
CH₂=C(R³)-C(O)-O-[CH₂CH(R⁴)O]ₘ -[CH(R⁵)-CH₂O]ₙ - R⁶
formule dans laquelle :
R3 est un atome d'hydrogène ou un radical méthyle,
R4 et R⁵ identiques ou différents, représentent un atome d'hydrogène ou un radical alkyle contenant de 1 à 4 atomes de carbone,
R⁶ est un radical alkyle, aryle, alkylaryle ou arylalkyle contenant de 1 à 30, de préférence de 8 à 30 atomes de carbone,
n est compris entre 2 et 100, de préférence entre 6 et 100 et m est compris entre 0 et 50, sous réserve que n soit supérieur ou égal à m et leur somme comprise entre 2 et 100, de préférence entre 6 et 100.

14. Granulés selon l'une des revendications précédentes, **caractérisés en ce que** le monomère (I) du polymère (i) ou (ii) est un acide monocarboxylique ou polycarboxylique, ou un anhydride carboxylique, est tel que l'un des radicaux R¹ est un atome d'hydrogène ; l'autre radical R¹ représente un atome d'hydrogène, un groupement -COOH ou un groupement - (CH₂)ₙ - COOH dans lequel n est compris entre 1 et 4, un radical alkyle en C₁-C₄ ; R'¹ représente un atome d'hydrogène, en groupement -(CH₂)ₘ - COOH dans lequel m est compris entre 1 et 4, un radical alkyle en C₁-C₄.

15. Granulés selon l'une quelconque des revendications 13 ou 14, **caractérisés en ce que** le monomère (I) du polymère (i) ou (ii) est choisi parmi les acides ou anhydrides acrylique, méthacrylique; citraconique maléique, fumarique, itaconique, crotonique.

16. Granulés selon l'une quelconque des revendications précédentes, **caractérisés en ce que** le monomère (II) est choisi parmi l'éthylène, le propylène, le 1-butène, l'isobutylène, le n-1-pentène, le 2-mëthyl 1-butène, le n-1-hexène, le 2-méthyl 1-pentène, le 4-méthyl 1-pentène, le 2-éthyl 1-butène, le diisobutylène (ou 2,4,4-triméthyl 1-pentène), le 2-méthyl 3,3-diméthyl 1-pentène.

17. Granulés selon l'une quelconque des revendications précédentes, **caractérisés en ce que** le monomère (III) est tel que R⁶ est un radical alkyle contenant de 8 à 30 atomes de carbone, un radical phényle substitué par un à trois groupements 1-phényléthyles, un radical alkylphényle dont le radical alkyle contient de 8 à 16 atomes de carbone.

18. Granulés selon l'une quelconque des revendications précédentes, **caractérisés en ce que** le greffon hydrophobe est choisi parmi les radicaux aliphatiques, cycliques, aromatiques, alkylaromatiques, arylaliphatiques, comprenant 4 à 30 atomes de carbone et pouvant être interrompus par un ou plusieurs hétéroatomes, de préférence l'oxygène.

19. Granulés selon l'une quelconque des revendications précédentes, **caractérisés en ce que** le polymère (i) est issu de la polymérisation de l'anhydride maléique et du 2,4,4 triméthyl-1-pentène.

20. Granulés selon l'une quelconque des revendications précédentes, **caractérisés en ce que** les polypeptides (iii) sont choisis parmi les polymères dérivés au moins de l'acide aspartique et/ou de l'acide glutamique.

21. Granulés selon l'une quelconque des revendications précédentes, **caractérisés en ce que** les polysaccharides (iv) sont choisis parmi les composés fortement dépolymérisés obtenus à partir de dextran, d'amidon, de maltodextrine, de gomme xanthane, et de galactomannanes, tels que le guar ou la caroube.

22. Granulés selon l'une quelconque des revendications précédentes, **caractérisés en ce que** la teneur en tensioactif polyalcoxylé non ionique et/ou en polymère polyalcoxylé amphiphile non ionique, présent dans la phase aqueuse externe, est comprise entre 1 et 10 % en poids par rapport au poids de l'émulsion inverse, de préférence entre 1 et 5 % en poids de l'émulsion inverse.

23. Granulés selon l'une des revendications précédentes, **caractérisés en ce que** la teneur en composé hydrosoluble ou hydrodispersable dans la phase aqueuse externe est telle que la teneur en ce composé dans le granulé final est comprise entre 30 et 85 % en poids par rapport au poids dudit granulé.

24. Granulés selon l'une des revendications précédentes, **caractérisés en ce que** le rapport pondéral émulsion inverse / phase aqueuse externe est compris entre 30/70 et 90/10, de préférence entre 50/50 et 90/10.

25. Granulés selon l'une des revendications précédentes, **caractérisés en ce que** les pressions osmotiques de la phase aqueuse externe et de la phase aqueuse interne sont équilibrées par ajout dans la phase aqueuse externe, d'au moins un additif choisi parmi les sels tels que les halogénures de métaux alcalins ou alcalino-terreux, d'au moins un sulfate de métal alcalin ou alcalino-terreux, parmi les sucres, ou encore parmi les polysaccharides, ou leurs mélanges.

26. Granulés selon l'une des revendications précédentes, **caractérisés en ce que** la phase aqueuse externe comprend une phase organique externe dispersée et/ou un solide dispersé.

27. Granulés selon la revendication précédente, **caractérisés en ce que** la phase organique externe dispersée représente 1 à 50 % en poids de la phase aqueuse externe, de préférence 5 à 25 % en poids de la phase aqueuse externe.

28. Granulés selon la revendication 26, **caractérisés en ce que** le solide dispersé représente 1 à 50 % en poids de la phase aqueuse externe, de préférence 5 à 25 % en poids de la phase aqueuse externe.

29. Granulés selon l'une quelconque des revendications précédentes, **caractérisés en ce que** la teneur en eau dans le granulé est comprise entre 10 et 50 % en poids du granulé, de préférence entre 20 et 30 %en poids du granulé.

30. Granulés selon l'une des revendications précédentes, **caractérisés en ce que** l'émulsion multiple est séchée par atomisation, ou en mettant en oeuvre des tambours Duprat^{®}, ou encore par lyophilisation.

## Patentansprüche

1. Granulat, das durch Trocknen einer inversen Emulsion, die in einer äußeren wässrigen Phase dispergiert ist, erhältlich ist, wobei durch das Trocknen die äußere wässrige Phase entfernt wird,
(a) wobei die inverse Emulsion eine innere wässrige Phase, die mindestens einen hydrophilen Wirkstoff, der in einer inneren organischen Phase dispergiert ist, umfasst, wobei die inverse Emulsion mindestens ein nichtionisches Tensid und/oder mindestens ein amphiphiles Blockpolymer und/oder mindestens ein kationisches Tensid umfasst;
(b) wobei die äußere wässrige Phase
- mindestens ein polyalkoxyliertes nichtionisches Tensid und/oder mindestens ein polyalkoxyliertes amphiphiles nichtionisches Polymer,
- mindestens ein wasserlösliches oder wasserdispergierbares Polymer, das in Gegenwart eines Wassergehalts von höchstens 10 Gew.-%, bezogen auf das Gewicht des Polymers, in fester Form vorliegt und eine Glasübergangstemperatur von mehr als 25°C, vorzugsweise mehr als 50°C, aufweist, umfasst.

2. Granulat nach Anspruch 1, **dadurch gekennzeichnet, dass** das nichtionische Tensid und das amphiphile Blockpolymer unter denjenigen ausgewählt sind, die gleichzeitig die nachstehenden zwei Bedingungen erfüllen:
- sie liegen dann, wenn sie mit der inneren organischen Phase in einer Konzentration zwischen 0,1 und 10 Gew.-% der Phase bei 25°C gemischt sind, in dem gesamten angegebenen Konzentrationsbereich oder einem Teil davon in Form einer Lösung vor;
- sie liegen dann, wenn sie mit der inneren wässrigen Phase in einer Konzentration zwischen 0,1 und 10 Gew.-% der Phase bei 25°C gemischt sind, in dem gesamten angegebenen Konzentrationsbereich oder einem Teil davon in Form einer Dispersion vor.

3. Granulat nach einem der vorhergehenden Ansprüche, **dadurch gekennzeichnet, dass** die Gesamtmenge an nichtionischem Tensid, amphiphilem Blockpolymer und/oder kationischem Tensid 2 bis 10 Gew.-% der inneren wässrigen Phase ausmacht.

4. Granulat nach einem der vorhergehenden Ansprüche, **dadurch gekennzeichnet, dass** die inverse Emulsion ein Gewichtsverhältnis von wässriger Phase zu organischer Phase zwischen 10/90 und 90/10, vorzugsweise zwischen 30/70 und 80/20, aufweist.

5. Granulat nach einem der vorhergehenden Ansprüche, **dadurch gekennzeichnet, dass** die innere wässrige Phase mindestens ein Additiv, das aus Salzen wie Alkali- oder Erdalkalimetallhalogeniden oder Alkali- oder Erdalkalimetallsulfaten, Zuckern oder Polysacchariden oder Mischungen davon ausgewählt ist, umfasst.

6. Granulat nach einem der vorhergehenden Ansprüche, **dadurch gekennzeichnet, dass** die Salzkonzentration in der inneren wässrigen Phase zwischen 0,05 und 1 mol/l liegt und vorzugsweise 0,1 bis 0,4 mol/l beträgt; die Zucker- und/oder Polysaccharidkonzentration derart beschaffen ist, dass der osmotische Druck der den Zucker und/oder das Polysaccharid umfassenden inneren wässrigen Phase dem osmotischen Druck einer inneren wässrigen Phase, die 0,05 bis 1 mol/l Salz umfasst, entspricht.

7. Granulat nach einem der vorhergehenden Ansprüche, **dadurch gekennzeichnet, dass** die innere organische Phase mindestens einen hydrophoben Wirkstoff, der mit dem in der inneren wässrigen Phase vorliegenden hydrophilen Wirkstoff verträglich ist, umfasst.

8. Granulat nach einem der vorhergehenden Ansprüche, **dadurch gekennzeichnet, dass** das Tensid und das Polymer, die in der äußeren wässrigen Phase vorliegen, unter denjenigen ausgewählt sind, die gleichzeitig die nachstehenden zwei Bedingungen erfüllen:
- sie liegen dann, wenn sie mit der äußeren wässrigen Phase in einer Konzentration zwischen 0,1 und 10 Gew.-% der Phase bei 25°C gemischt sind, in dem gesamten angegebenen Konzentrationsbereich oder einem Teil davon in Form einer Lösung vor;
- sie liegen dann, wenn sie mit der inneren organischen Phase in einer Konzentration zwischen 0,1 und 10 Gew.-% der Phase bei 25°C gemischt sind, in dem gesamten angegebenen Konzentrationsbereich oder einem Teil davon in Form einer Dispersion vor.

9. Granulat nach einem der vorhergehenden Ansprüche, **dadurch gekennzeichnet, dass** die äußere wässrige Phase mindestens ein thermoverdickendes Polymer umfasst.

10. Granulat nach einem der vorhergehenden Ansprüche, **dadurch gekennzeichnet, dass** das thermoverdickende Polymer aus Polymeren mit einem derartigen Viskositätssprung zwischen 25°C und 80°C, dass der Wert des Verhältnisses log₁₀ (Viskosität bei 80°C) /log₁₀ (Viskosität bei 25°C) mindestens gleich 1, vorzugsweise mindestens gleich 2, ist, und aus denen, bei denen die Viskositätsänderung reversibel ist, ausgewählt ist.

11. Granulat nach einem der Ansprüche 9 oder 10, **dadurch gekennzeichnet, dass** der Gehalt an thermoverdickendem Polymer zwischen 0,2 und 10 Gew.-% der äußeren wässrigen Phase, vorzugsweise zwischen 1 und 5 Gew.-% der äußeren wässrigen Phase, liegt.

12. Granulat nach dem vorhergehenden Anspruch, **dadurch gekennzeichnet, dass** das wasserlösliche oder wasserdispergierbare Polymer aus
(i) mindestens einem Polymer, erhalten durch Polymerisation
∘ von mindestens einem aliphatischen, cyclischen oder aromatischen, linearen oder verzweigten, ethylenisch ungesättigten Mono- oder Polycarbonsäure- oder Anhydridmonomer (I) und
∘ mindestens einem linearen oder verzweigten, monoethylenisch oder polyethylenisch ungesättigten Kohlenwasserstoffmonomer und/oder
∘ mindestens einem ethylenisch ungesättigten polyalkoxylierten Carbonsäureestermonomer (III);
(ii) mindestens einem Polymer, das aus der Polymerisation von mindestens einem aliphatischen, cyclischen oder aromatischen, linearen oder verzweigten, ethylenisch ungesättigten Mono- oder Polycarbonsäure- oder Anhydridmonomer (I) stammt und gegebenenfalls mindestens einen gesättigten oder ungesättigten, aromatischen oder nichtaromatischen, gegebenenfalls durch ein oder mehrere Heteroatome unterbrochenen hydrophoben C₄-C₃₀-Kohlenwasserstoffpfropfanteil umfasst;
(iii) Proteinen und Polypeptiden natürlicher oder synthetischer Herkunft, die gegebenenfalls mindestens einen gesättigten oder ungesättigten, aromatischen oder nichtaromatischen, gegebenenfalls durch ein oder mehrere Heteroatome unterbrochenen hydrophoben C₄-C₃₀-Kohlenwasserstoffpfropfanteil umfassen;
(iv) Polysacchariden, die vorzugsweise stark depolymerisiert sind und mindestens einen gesättigten oder ungesättigten, aromatischen oder nichtaromatischen, gegebenenfalls durch ein oder mehrere Heteroatome unterbrochenen hydrophoben C₄-C₃₀-Kohlenwasserstoffpfropfanteil umfassen;
(v) Polyvinylalkohol, Polyvinylpyrrolidon ausgewählt ist.

13. Granulat nach einem der vorhergehenden Ansprüche, **dadurch gekennzeichnet, dass** es sich bei der in der äußeren wässrigen Phase vorliegenden wasserlöslichen oder wasserdispergierbaren Verbindung um ein Polymer (i), das aus der Polymerisation
∘ von mindestens einem Monomer der Formel (I):
(R¹) (R¹)-C=C(R'¹)-COOH (I)
worin die Reste R¹ und R'¹ gleich oder verschieden sind und für ein Wasserstoffatom, einen C₁-C₁₀-Kohlenwasserstoffrest, der gegebenenfalls eine -COOH-Gruppe umfasst, oder eine -COOH-Gruppe stehen; und
∘ von mindestens einem Monomer der Formel (II):
(R²) (R²)-CH₂ (II)
worin die Reste R² gleich oder verschieden sind und für einen linearen oder verzweigten C₁-C₁₀-Kohlenwasserstoffrest stehen; und/oder
∘ von mindestens einem Monomer der Formel (III):
CH₂=C(R³) -C (O) -O-[CH₂CH (R⁴) O]ₘ-[CH(R⁵) -CH₂O]ₙ-R⁶
worin
R³ für ein Wasserstoffatom oder einen Methylrest steht,
R⁴ und R⁵ gleich oder verschieden sind und für ein Wasserstoffatom oder einen Alkylrest mit 1 bis 4 Kohlenstoffatomen stehen,
R⁶ für einen Alkyl-, Aryl-, Alkylaryl- oder Arylalkylrest mit 1 bis 30 Kohlenstoffatomen und vorzugsweise 8 bis 30 Kohlenstoffatomen steht,
n zwischen 2 und 100 und vorzugsweise zwischen 6 und 100 liegt und m zwischen 0 und 50 liegt, mit der Maßgabe, dass n größer gleich m ist und ihre Summe zwischen 2 und 100 und vorzugsweise zwischen 6 und 100 liegt;
stammt, handelt.

14. Granulat nach einem der vorhergehenden Ansprüche, **dadurch gekennzeichnet, dass** das Monomer (I) des Polymers (i) oder (ii) eine Mono- oder Polycarbonsäure oder ein Carbonsäureanhydrid ist und derart beschaffen ist, dass einer der Reste R¹ für ein Wasserstoffatom steht; der andere Rest R¹ für ein Wasserstoffatom, eine -COOH-Gruppe, eine -(CH₂)ₙ-COOH-Gruppe, worin n zwischen 1 und 4 liegt, oder einen C₁-C₄-Alkylrest steht und R'¹ für ein Wasserstoffatom, eine -(CH₂)ₘ-COOH-Gruppe, worin m zwischen 1 und 4 liegt, oder einen C₁-C₄-Alkylrest steht.

15. Granulat nach einem der Ansprüche 13 oder 14, **dadurch gekennzeichnet, dass** das Monomer (I) des Polymers (i) oder (ii) aus Acrylsäure, Methacrylsäure, Citraconsäure, Maleinsäure, Fumarsäure, Itaconsäure, Crotonsäure und deren Anhydriden ausgewählt ist.

16. Granulat nach einem der vorhergehenden Ansprüche, **dadurch gekennzeichnet, dass** das Monomer (II) aus Ethylen, Propylen, 1-Buten, Isobutylen, n-1-Penten, 2-Methyl-1-buten, n-1-Hexen, 2-Methyl-1-penten, 4-Methyl-1-penten, 2-Ethyl-1-buten, Diisobutylen (oder 2,4,4-Trimethyl-1-penten) und 2-Methyl-3,3-dimethyl-1-penten ausgewählt ist.

17. Granulat nach einem der vorhergehenden Ansprüche, **dadurch gekennzeichnet, dass** das Monomer (III) derart beschaffen ist, dass R⁶ für einen Alkylrest mit 8 bis 30 Kohlenstoffatomen, einen Phenylrest, der durch eine bis drei 1-Phenylethylgruppen substituiert ist, oder einen Alkylphenylrest mit 8 bis 16 Kohlenstoffatomen im Alkylrest steht.

18. Granulat nach einem der vorhergehenden Ansprüche, **dadurch gekennzeichnet, dass** der hydrophobe Pfropfanteil aus aliphatischen, cyclischen, aromatischen, alkylaromatischen und arylaliphatischen Resten mit 4 bis 30 Kohlenstoffatomen, die gegebenenfalls durch ein oder mehrere Heteroatome, vorzugsweise Sauerstoff, unterbrochen sein können, ausgewählt ist.

19. Granulat nach einem der vorhergehenden Ansprüche, **dadurch gekennzeichnet, dass** das Polymer (i) aus der Polymerisation von Maleinsäureanhydrid und 2,4,4-Trimethyl-1-penten stammt.

20. Granulat nach einem der vorhergehenden Ansprüche, **dadurch gekennzeichnet, dass** die Polypeptide (iii) aus Polymeren, die sich mindestens von Asparaginsäure und/oder Glutaminsäure ableiten, ausgewählt sind.

21. Granulat nach einem der vorhergehenden Ansprüche, **dadurch gekennzeichnet, dass** die Polysaccharide (iv) aus stark depolymerisierten Verbindungen, die aus Dextran, Stärke, Maltodextrin, Xanthan und Galactomannanen, wie Guar oder Johannisbrot, erhalten werden, ausgewählt sind.

22. Granulat nach einem der vorhergehenden Ansprüche, **dadurch gekennzeichnet, dass** der Gehalt an polyalkoxyliertem nichtionischem Tensid und/oder polyalkoxyliertem amphiphilem nichtionischem Polymer in der äußeren wässrigen Phase zwischen 1 und 10 Gew.-%, bezogen auf das Gewicht der inversen Emulsion, vorzugsweise zwischen 1 und 5 Gew.-% der inversen Emulsion, liegt.

23. Granulat nach einem der vorhergehenden Ansprüche, **dadurch gekennzeichnet, dass** der Gehalt an wasserlöslicher oder wasserdispergierbarer Verbindung in der äußeren wässrigen Phase derart beschaffen ist, dass der Gehalt an dieser Verbindung in dem fertigen Granulat zwischen 30 und 85 Gew.-%, bezogen auf das Gewicht des Granulats, liegt.

24. Granulat nach einem der vorhergehenden Ansprüche, **dadurch gekennzeichnet, dass** das Gewichtsverhältnis von inverser Emulsion zu äußerer wässriger Phase zwischen 30/70 und 90/10 und vorzugsweise zwischen 50/50 und 90/10 liegt.

25. Granulat nach einem der vorhergehenden Ansprüche, **dadurch gekennzeichnet, dass** die osmotischen Drücke der äußeren wässrigen Phase und der inneren wässrigen Phase durch Zusatz mindestens eines Additivs in der äußeren wässrigen Phase, das aus Salzen wie Alkali- oder Erdalkalimetallhalogeniden, mindestens einem Alkali- oder Erdalkalimetallsulfat, Zuckern oder auch Polysacchariden oder Mischungen davon ausgewählt ist, äquilibriert sind.

26. Granulat nach einem der vorhergehenden Ansprüche, **dadurch gekennzeichnet, dass** die äußere wässrige Phase eine dispergierte äußere organische Phase und/oder einen dispergierten Feststoff umfasst.

27. Granulat nach dem vorhergehenden Anspruch, **dadurch gekennzeichnet, dass** die dispergierte äußere organische Phase 1 bis 50 Gew.-% der äußeren wässrigen Phase und vorzugsweise 5 bis 25 Gew.-% der äußeren wässrigen Phase ausmacht.

28. Granulat nach Anspruch 26, **dadurch gekennzeichnet, dass** der dispergierte Feststoff 1 bis 50 Gew.-% der äußeren wässrigen Phase und vorzugsweise 5 bis 25 Gew.-% der äußeren wässrigen Phase ausmacht.

29. Granulat nach einem der vorhergehenden Ansprüche, **dadurch gekennzeichnet, dass** der Wassergehalt in dem Granulat zwischen 10 und 50 Gew.-% des Granulats und vorzugsweise zwischen 20 und 30 Gew.-% des Granulats liegt.

30. Granulat nach einem der vorhergehenden Ansprüche, **dadurch gekennzeichnet, dass** die multiple Emulsion durch Sprühtrocknung oder unter Verwendung von Duprat™-Trommeln oder durch Gefriertrocknung getrocknet wird.

## Claims

1. Granules which may be obtained by drying an inverse emulsion, dispersed in an external aqueous phase, said drying operation removing the external aqueous phase,
(a) the inverse emulsion comprising an internal aqueous phase comprising at least one hydrophilic active substance, dispersed in an internal organic phase, said inverse emulsion comprising at least one nonionic surfactant and/or at least one amphiphilic block polymer and/or at least one cationic surfactant;
(b) the external aqueous phase comprising:
- at least one polyalkoxylated nonionic surfactant and/or at least one polyalkoxylated amphiphilic nonionic polymer,
- at least one water-soluble or water-dispersible polymer provided in a solid form in the presence of a water content of at most 10% by weight relative to the weight of said polymer and whose glass transition temperature is greater than 25°C, preferably greater than 50°C.

2. Granules according to Claim 1, **characterized in that** the nonionic surfactant and the amphiphilic block polymer are chosen from those which satisfy both of the two conditions below:
- when they are mixed with the internal organic phase, at a concentration between 0.1 and 10% by weight of said phase at 25°C, they are in the form of a solution in the whole or part of the concentration range indicated;
- when they are mixed with the internal aqueous phase, at a concentration between 0.1 and 10% by weight of said phase and at 25°C, they are in the form of a dispersion in the whole or part of the concentration range indicated.

3. Granules according to either of the preceding claims, **characterized in that** the total quantity of nonionic surfactant, of amphiphilic block polymer and/or of cationic surfactant represents 2 to 10% by weight of the internal aqueous phase.

4. Granules according to one of the preceding claims, **characterized in that** the inverse emulsion has an aqueous phase/organic phase weight ratio between 10/90 and 90/10, preferably between 30/70 and 80/20.

5. Granules according to one of the preceding claims, **characterized in that** the internal aqueous phase comprises at least one additive chosen from salts such as alkali or alkaline-earth metal halides, or alkali or alkaline-earth metal sulfates, from sugars, or from polysaccharides, or mixtures thereof.

6. Granules according to the preceding claim, **characterized in that** the concentration of salt in the internal aqueous phase is between 0.05 and 1 mol/l, preferably 0.1 to 0.4 mol/l; the concentration of sugar and/or polysaccharide is such that the osmotic pressure of the internal aqueous phase comprising the sugar and/or the polysaccharide corresponds to the osmotic pressure of an internal aqueous phase comprising 0.05 to 1 mol/l of salt.

7. Granules according to one of the preceding claims, **characterized in that** the internal organic phase comprises at least one hydrophobic active substance, compatible with the hydrophilic active substance present in the internal aqueous phase.

8. Granules according to one of the preceding claims, **characterized in that** the surfactant and the polymer present in the external aqueous phase are chosen from those which satisfy both of the two conditions below:
- when they are mixed with the external aqueous phase, at a concentration of between 0.1 and 10% by weight of said phase at 25°C, they exist in the form of a solution in the whole or part of the concentration range indicated;
- when they are mixed with the internal organic phase, at a concentration between 0.1 and 10% by weight of said phase and at 25°C, they exist in the form of a dispersion in the whole or part of the concentration range indicated.

9. Granules according to one of the preceding claims, **characterized in that** the external aqueous phase comprises at least one thermothickening polymer.

10. Granules according to one of the preceding claims, **characterized in that** the thermothickening polymer is chosen from polymers exhibiting a jump in viscosity between 25 and 80°C such that the value of the log₁₀(viscosity at 80°C)/log₁₀(viscosity at 25°C) ratio is at least equal to 1, preferably at least equal to 2, and from those in which the variation of viscosity is reversible.

11. Granules according to either of the preceding Claims 9 and 10, **characterized in that** the content of thermothickening polymer is between 0.2 and 10% by weight of the external aqueous phase, preferably between 1 and 5% by weight of the external aqueous phase.

12. Granules according to the preceding claim, **characterized in that** the water-soluble or water-dispersible polymer is chosen from:
(i) at least one polymer obtained by polymerization
∘ of at least one monomer (I) which is an ethylenically unsaturated, linear or branched, aliphatic, cyclic or aromatic monocarboxylic or polycarboxylic acid, or anhydride, and
∘ of at least one monoethylenically or polyethylenically unsaturated, linear or branched hydrocarbon monomer, and/or
∘ of at least one monomer (III) which is a polyalkoxylated ester of ethylenically unsaturated carboxylic acid;
(ii) at least one polymer obtained from the polymerization of at least one monomer (I) which is an ethylenically unsaturated, linear or branched, aliphatic, cyclic or aromatic, monocarboxylic or polycarboxylic acid, or anhydride, and optionally comprising at least one saturated or unsaturated, aromatic or nonaromatic, hydrophobic C₄-C₃₀ hydrocarbon graft, optionally interrupted by one or more heteroatoms;
(iii) proteins and polypeptides of natural or synthetic origin, optionally comprising at least one saturated or unsaturated, aromatic or nonaromatic, hydrophobic C₄-C₃₀ hydrocarbon graft, optionally interrupted by one or more heteroatoms;
(iv) polysaccharides, preferably highly depolymerized, optionally containing at least one saturated or unsaturated, aromatic or nonaromatic, hydrophobic C₄-C₃₀ hydrocarbon graft, optionally interrupted by one or more heteroatoms;
(v) polyvinyl alcohol, polyvinylpyrrolidone.

13. Granules according to one of the preceding claims, **characterized in that** the water-soluble or water-dispersible compound present in the external aqueous phase is a polymer (i) obtained from the polymerization
∘ of at least one monomer of formula (I):
(R¹)(R¹)-C= C(R'¹)-COOH (I)
in which formula the radicals R¹, R'¹, which are identical or different, represent a hydrogen atom, a C₁-C₁₀ hydrocarbon radical optionally comprising a -COOH group, a -COOH group; and
∘ of at least one monomer of formula (II):
(R²) (R²) -C = CH₂ (II)
in which formula the radicals R², which are identical or different, represent a linear or branched C₁-C₁₀ hydrocarbon radical; and/or
∘ of at least one monomer of formula (III):
CH₂=C(R³)-C(O) -O-[CH₂CH(R⁴)O]ₘ-[CH(R⁵) -CH₂O]ₙ-R⁶
in which formula:
R³ is a hydrogen atom or a methyl radical,
R⁴ and R⁵ which are identical or different, represent a hydrogen atom or an alkyl radical containing from 1 to 4 carbon atoms,
R⁶ is an alkyl, aryl, alkylaryl or arylalkyl radical containing from 1 to 30, preferably from 8 to 30 carbon atoms,
n is between 2 and 100, preferably between 6 and 100 and m is between 0 and 50, provided that n is greater than or equal to m and their sum is between 2 and 100, preferably between 6 and 100.

14. Granules according to one of the preceding claims, **characterized in that** the monomer (I) of the polymer (i) or (ii) is a monocarboxylic or polycarboxylic acid, or a carboxylic anhydride, is such that one of the radicals R¹ is a hydrogen atom; the other radical R¹ represents a hydrogen atom, a -COOH group or a -(CH₂)ₙ-COOH group in which n is between 1 and 4, a C₁-C₄ alkyl radical; R'¹ represents a hydrogen atom, as a -(CH₂)ₘ-COOH group in which m is between 1 and 4, a C₁-C₄ alkyl radical.

15. Granules according to either of Claims 13 and 14, **characterized in that** the monomer (I) of the polymer (i) or (ii) is chosen from acrylic, methacrylic, citraconic, maleic, fumaric, itaconic and crotonic acids or anhydrides.

16. Granules according to any one of the preceding claims, **characterized in that** the monomer (II) is chosen from ethylene, propylene, 1-butene, isobutylene, n-1-pentene, 2-methyl-1-butene, n-1-hexene, 2-methyl-1-pentene, 4-methyl-1-pentene, 2-ethyl-1-butene, diisobutylene (or 2,4,4-trimethyl-1-pentene), 2-methyl-3,3-dimethyl-1-pentene.

17. Granules according to any one of the preceding claims, **characterized in that** the monomer (III) is such that R⁶ is an alkyl radical containing from 8 to 30 carbon atoms, a phenyl radical substituted with one to three 1-phenylethyl groups, an alkylphenyl radical in which the alkyl radical contains from 8 to 16 carbon atoms.

18. Granules according to any one of the preceding claims, **characterized in that** the hydrophobic graft is chosen from aliphatic, cyclic, aromatic, alkylaromatic and arylaliphatic radicals comprising 4 to 30 carbon atoms, and which may be interrupted by one or more heteroatoms, preferably oxygen.

19. Granules according to any one of the preceding claims, **characterized in that** the polymer (i) is obtained from the polymerization of maleic anhydride and 2,4,4-trimethyl-1-pentene.

20. Granules according to any one of the preceding claims, **characterized in that** the polypeptides (iii) are chosen from the polymers derived from at least aspartic acid and/or glutamic acid.

21. Granules according to any one of the preceding claims, **characterized in that** the polysaccharides (iv) are chosen from the highly depolymerized compounds obtained from dextran, starch, maltodextrin, xanthan gum, and galactomannans, such as guar or carob.

22. Granules according to any one of the preceding claims, **characterized in that** the content of nonionic polyalkoxylated surfactant and/or of nonionic amphiphilic polyalkoxylated polymer, present in the external aqueous phase, is between 1 and 10% by weight relative to the weight of the inverse emulsion, preferably between 1 and 5% by weight of the inverse emulsion.

23. Granules according to one of the preceding claims, **characterized in that** the content of water-soluble or water-dispersible compound in the external aqueous phase is such that the content of this compound in the final granule is between 30 and 85% by weight relative to the weight of said granule.

24. Granules according to one of the preceding claims, **characterized in that** the inverse emulsion/external aqueous phase weight ratio is between 30/70 and 90/10, preferably between 50/50 and 90/10.

25. Granules according to one of the preceding claims, **characterized in that** the osmotic pressures of the external aqueous phase and of the internal aqueous phase are equilibrated by adding to the external aqueous phase at least one additive chosen from salts such as alkali or alkaline-earth metal halides, at least one alkali or alkaline-earth metal sulfate, from sugars, or from polysaccharides, or mixtures thereof.

26. Granules according to one of the preceding claims, **characterized in that** the external aqueous phase comprises a dispersed external organic phase and/or a dispersed solid.

27. Granules according to the preceding claim, **characterized in that** the dispersed external organic phase represents 1 to 50% by weight of the external aqueous phase, preferably 5 to 25% by weight of the external aqueous phase.

28. Granules according to Claim 26, **characterized in that** the dispersed solid represents 1 to 50% by weight of the external aqueous phase, preferably 5 to 25% by weight of the external aqueous phase.

29. Granules according to any one of the preceding claims, **characterized in that** the water content in the granule is between 10 and 50% by weight of the granule, preferably between 20 and 30% by weight of the granule.

30. Granules according to one of the preceding claims, **characterized in that** the multiple emulsion is dried by spray-drying, or using Duprat® drums, or by freeze-drying.
